(19) **Europäisches Patentamt**

**European Patent Office**

**Office européen des brevets**

(11) **EP 1 283 061 A1**

(12) **EUROPEAN PATENT APPLICATION**
published in accordance with Art. 158(3) EPC

(43) Date of publication:
**12.02.2003 Bulletin 2003/07**

(51) Int Cl.[7]: **A61L 2/20**, A61L 2/26, B65B 55/04

(21) Application number: **01930156.3**

(22) Date of filing: **15.05.2001**

(86) International application number:
**PCT/JP01/04039**

(87) International publication number:
**WO 01/087359 (22.11.2001 Gazette 2001/47)**

(84) Designated Contracting States:
**AT BE CH CY DE DK ES FI FR GB GR IE IT LI LU MC NL PT SE TR**

(30) Priority: **16.05.2000 JP 2000143481**

(71) Applicant: **Taisei Kako Co., Ltd.
Osaka-shi, Osaka 531-0073 (JP)**

(72) Inventors:
• **YAMAMOTO, Yuichi, c/o TAISEI KAKO CO., LTD
Ibaraki-shi, Osaka 567-0054 (JP)**

• **NAKAMURA, Koichi, c/o TAISEI KAKO CO., LTD
Ibaraki-shi, Osaka 567-0054 (JP)**
• **NONAKA, Toshinori, c/o TAISEI KAKO CO., LTD
Ibaraki-shi, Osaka 567-0054 (JP)**

(74) Representative: **HOFFMANN - EITLE
Patent- und Rechtsanwälte
Arabellastrasse 4
81925 München (DE)**

(54) **METHOD FOR MANUFACTURING SEALING TOOL STERILIZED WITH GASEOUS HYDROGEN PEROXIDE**

(57) It is an object to establish a sterilization condition and means for decreasing the amount of a remaining sterilization agent as much as possible after sterilizing a rubbery sealing tool. In a sterilizing method using gaseous hydrogen peroxide, a filling rate of a sample in a sterilization bag for a normal type is set to 45 % or more and a sterilization condition is set to a hydrogen peroxide treatment of 3 pulses and an aeration pulse of 20 pulses, thereby carrying out a sterilization treatment. Furthermore, it is preferable that a volume rate in a bag of an outer bag accommodating the sterilization bag should be set to 55 to 12 %. By such setting, a survival ratio of 0/10 of a bacteria cell could be realized at first to fifth times of repetition of the sterilization treatment.

*Fig. 1*

**Description**

Technical Field

The present invention relates to a method for the production of a variety of sealing tools sterilized with gaseous hydrogen peroxide. More specifically, the present invention pertains to a method for the production of a sterilized sealing tool, which comprises the step of sterilizing a sealing tool such as a cap, a gasket for a piston or a plunger head inserted into an injection cylinder such as a syringe, a tool for preventing liquid leakage such as rubber boots and an elastic ring for a bushing or for fitting a joint such as an O-ring, with hydrogen peroxide vapor, a hydrogen peroxide-containing gas, or an aerosol of hydrogen peroxide obtained by, for instance, the atomization of a hydrogen peroxide solution.

Background Art

Conventionally, the sterilization of, in particular, machinery and tools for medical use have been carried out, for a long time, according to the dry sterilization and the sterilization by boiling in hot water. Thereafter, the level of the sterilization has been further improved because of the development of the pressurized steam-sterilizing device. In this connection, there have been developed and put into practical use the ethylene oxide gas (hereunder abbreviated as "EOG") sterilization, the γ-ray-irradiation sterilization and the electron beam-irradiation sterilization for treating the articles to be processed, to which none of the foregoing sterilization methods can easily be applied.

In this regard, the treatment with ethylene oxide gas (EOG) is advantageous in that the gas can penetrate even into fine spaces of articles to be processed, but it has been found that this treatment suffers from the following problems:

First, a considerable amount of the sterilization agent and, for instance, ethylene glycol (hereunder abbreviated as "EG") and/or ethylene chlorohydrin (hereunder abbreviated as "ECH") derived from the sterilization agent remain on the articles thus treated even after the sterilization treatment and as a result, a serious problem has been indicated such that this would be involved in the production of cancer.

Moreover, the γ-ray-irradiation sterilization requires neither the exposure of articles to be processed to any high temperature nor the drying thereof after being wetted with a liquid and is excellent in that this treatment permits the sterilization of an article having a complicated shape and even the shaded portions thereof while making the most use of the high penetrability and that the γ-rays per se is colorless, odorless and tasteless. The electron beam (cathode rays) has a penetrability inferior to that of the γ-rays, but the sterilizing power thereof while making use of the ability of ionizing the object to be treated has sufficiently been highly estimated.

However, the γ-rays and the electron beams belong to the high energy radioactive rays and for this reason, the practical use thereof has been restricted since the irradiation of an article with γ-rays or electron beams may easily deteriorate the surface area or the like of the article and a personnel who handles them must be authorized by the license for the licensed engineer of radiation.

Japanese Un-Examined Patent Publication No. Hei 10-24093 (prior reference 1) discloses a sterilization device, which makes use of gaseous hydrogen peroxide and a sterilization treatment, which makes use of a combination of three members: a transfer box, a sterilization chamber and a sterilized container.

This prior reference 1 discloses in the section entitled "Technical Field of the Invention" as follows: the present invention relates to a sterilization device and more particularly to a sterilization device for sterilizing the surface of an article, for instance, a small-sized container from a synthetic resin such as a container for ophthalmic medicaments or other small-sized molded articles from synthetic resins.

In addition, the prior reference 1 cites, in the section "[0004]", Japanese Examined Patent Publication No. Sho 60-8826 as a prior art technique for this invention, refers to the following passage from the section of the reference entitled "Method for Washing, Sterilizing and Drying Rubber Stoppers for Vials": the following steps are successively conducted: a degassing step, a washing step, a foreign substance-eluting step, a rinsing and washing step and a highly pressurized steam-sterilization step in which highly pressurized steam maintained at a temperature and pressure at which the rubber stoppers for vials are not deteriorated is introduced into a steam sterilizer to thus sterilize the rubber stoppers for vials and thus indicates that the pressurized steam-sterilization of "the rubber stoppers for vials" is conducted in the invention of the foregoing cited reference.

However, the prior reference 1 does not directly disclose a method for sterilizing "the rubber stoppers for vials" with gaseous hydrogen peroxide. More specifically, the prior reference 1 neither refers to nor suggests any specific method for sterilizing rubber stoppers (for vials) or rubber caps with gaseous hydrogen peroxide and specific conditions for the sterilization, but the prior reference simply and specifically describes, in the latter half of the specification, the sterilization device using "containers for ophthalmic medicaments".

As a sterilization technique, which has attracted special interest lately, there may be listed, for instance, a gas-irradiation combined method in which gaseous hydrogen peroxide is injected under a high vacuum (about 0.04 kPa) and thereafter an object is irradiated with plasma beams (charged corpuscular flow).

[Problems That the Invention is to Solve]

A variety of the foregoing conventional techniques may permit the sterilization of microorganisms attached just to the surface of an article to be processed, with difficulty, but this technique cannot sufficiently kill the microorganisms penetrating into the slightly interior region of the article to be processed. More specifically, there are, in fact, many fine and uneven portions on the surface of a sealing tool such as a rubber stopper and therefore, microorganisms inevitably penetrate into these fine concave portions of the article. However, the conventional sterilization methods using gaseous hydrogen peroxide or hydrogen peroxide vapor cannot kill the microorganisms concealed in these fine concave portions of the article to such an extent that the presence of microorganisms can be neglected.

The present invention is rather an improved technique of those disclosed in the foregoing prior references 1, 2 and 3, completed by investigating the scope of application thereof on the basis of these prior references. However, the purpose of the present invention is to provide a specific sterilization procedure, a sterilizing condition and the like which use gaseous hydrogen peroxide when applying, to a rubber gasket and other sealing tools, an effective sterilizing method and sterilizing condition which have been neither described nor suggested in any of the prior references. More specifically, it is a purpose of the present invention to provide a method for sterilizing a sealing tool and a sealing tool sterilized using the method. As a result of serious investigations of the procedure and condition and the like, the present inventor completed the present invention.

[Means for Solving the Problems]

According to the present invention, the desired effects or intended purposes of the present invention can be accomplished by combining the following essential elements:

(1) A method for the production of a sealing tool for setting a filling rate in a sterilization bag for a sample to be sterilized to 45 % or more for a gasket for a normal type syringe and to 20 % or more for a gasket for a large-sized syringe, thereby carrying out sterilization in the case in which a rubbery sealing tool or a sealing tool made from an olefinic resin is held as an article to be processed in a sterilization unit under a high vacuum, gaseous hydrogen peroxide is then introduced into the sterilization unit, and is held for a predetermined time and is sterilized by at least one member selected from the group consisting of active oxygen and radial hydroxide, a clean gas is thereafter introduced and is held for a predetermined time to cause a sterilizing substance to penetrate into an inner side of the article to be processed, thereby setting a sterilizing condition using the gaseous hydrogen peroxide in which a sterilization treatment for the article to be processed is one sterilization pulse to a combination of 70 (g/pulse) of an aqueous hydrogen peroxide solution (a concentration of 35 % by weight) injection amount X 3 pulses and 20 pulses of an aeration. An aqueous hydrogen peroxide solution (normally a concentration of 35 % by weight) is injected in a predetermined amount [for example, 70 (g/pulse)] into a hydrogen peroxide gas generating device, and is changed into gaseous hydrogen peroxide in the generating device and is introduced into a sterilizer (or a sterilization chamber) through a supply conduit, and then comes in contact with a sample. Thus, the gaseous hydrogen peroxide fulfills the function of oxidizing and sterilizing the sample.

(2) The method for the production of a sealing tool as set forth in the foregoing item 1, wherein the filling rate of the sample to be sterilized which is accommodated in the sterilization bag is set to 50 % or more for a gasket for a normal type syringe and to 20 % or more for a gasket for a large-sized syringe under the sterilization condition, thereby carrying out the sterilization.

(3) A method for the production of a sealing tool, wherein in the case in which a sterilization condition is set to a combination of 70 (g/pulse) of an injected amount of an aqueous hydrogen peroxide solution X 4 pulses and 20 pulses of an aeration, a filling rate of a sample to be sterilized in a sterilization bag is set to 20 % or more for a gasket for a normal type syringe or a gasket for a large-sized syringe, thereby carrying out the sterilization.

(4) The method for the production of a sealing tool as set forth in any one of the foregoing items 1 to 3, wherein the number of repetitions of the aeration pulse to be carried out next to a sterilization pulse in the sterilization treatment is 30 pulses or more.

(5) The method for the production of a sealing tool as set forth in any one of the foregoing items 1 to 3, wherein the number of repetitions of the aeration pulse is 5 to 50 pulses or more.

(6) The method for the production of a sealing tool as set forth in any one of the foregoing items 1 to 4, wherein the sterilization pulses and the aeration pulses are, in advance, conducted in combination.

(7) The method for the production of a sealing tool as set forth in any one of the foregoing items 1 to 5, wherein an outer bag further accommodating the sterilization bag having the article to be processed is mounted in a porous container for mounting with a volume rate of 12 to 55 %, thereby carrying out the sterilization treatment.

(8) The method for the production of a sealing tool as set forth in any one of the foregoing items 1 to 5, wherein the article to be processed is at least one member selected from a rubber cap, a rubber gasket, a gasket for a piston (plunger) to be inserted into an injection cylinder (syringe), a tool for preventing liquid leakage such as rubber boots, and an elastic ring for a bushing and for fitting a joint.

(9) The method for the production of a sealing member as set forth in any one of the foregoing items 1 to 7, wherein rubber is at least one member selected from the following conjugated diene rubber and non-conjugated diene rubber: the conjugated diene rubber being natural rubber, a variety of synthetic rubber materials, blends of each comprising at least two of these natural and synthetic rubber materials and copolymer rubber comprising repeating units of these rubber materials and other repeating units copolymerizable therewith, wherein the synthetic rubber comprises 1,4-cis-polyisoprene rubber obtained by 1,4-addition polymerization of isoprene, which is a repeating unit mainly constituting the natural rubber, 1,4-cis-polybutadiene rubber and isobutene-isoprene copolymer rubber; the non-conjugated diene rubber being copolymer rubber materials of at least two 1-olefins or multi-component copolymer rubber materials obtained by copolymerizing these monomers with third non-conjugated dienes, wherein the copolymer rubber materials of at least two 1-olefins is at least one member selected from the group consisting of ethylene-propylene (copolymer) rubber, ethylene-1-butene copolymer rubber and propylene-1-butene copolymer rubber, and wherein the multi-component copolymer rubber obtained by copolymerizing these monomers with a third non-conjugated diene is at least one member selected from the group consisting of ethylene-propylene-1,4-hexadiene copolymer rubber, ethylene-propylene-methylene norbornene copolymer rubber and ethylene-propylene-ethylidene norbornene copolymer rubber.

(10) The method for the production of a sealing tool as set forth in any one of items 1 to 8, wherein the thermoplastic elastomer (thermoplastic rubber) is a polymer or a kneaded composition (kneaded mixture) of at least two polymers, which simultaneously has characteristic properties of thermoplastic resin and elastomer; the polymer composition, which can be formed into a variety of shapes as set forth in the molding method applicable to the resin and can be subjected to vulcanization treatment (crosslinking treatment) applicable to the elastomer, is at least one kneaded composition selected from the group consisting of kneaded compositions of polyolefin resins and ethylene-propylene (copolymer) rubber, kneaded compositions of polyolefin resins and ethylene-propylene-non-conjugated diene copolymer rubber and kneaded compositions of propylene-1-butene copolymer resins and ethylene-propylene-non-conjugated diene copolymer rubber.

(11) The method for the production of a sealing tool as set forth in any one of items 1 to 9, wherein the thermoplastic elastomer is a thermally kneaded composition comprising at least one member selected from the group consisting of polyethylene resins, polypropylene resins, poly-1-butene resins, poly-4-methyl-1-pentene resin and poly-1-hexene resins; and at least one member selected from the group consisting of ethylene-propylene-1,4-hexadiene copolymer rubber, ethylene-propylene-methylene norbornene copolymer rubber and ethylene-propylene-ethylidene norbornene copolymer rubber.

[Brief Description of the Drawings]

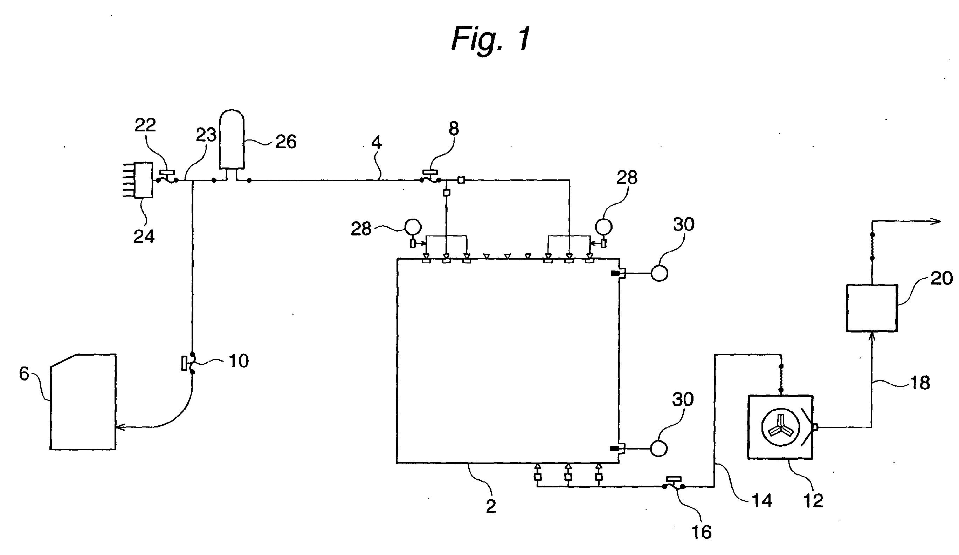

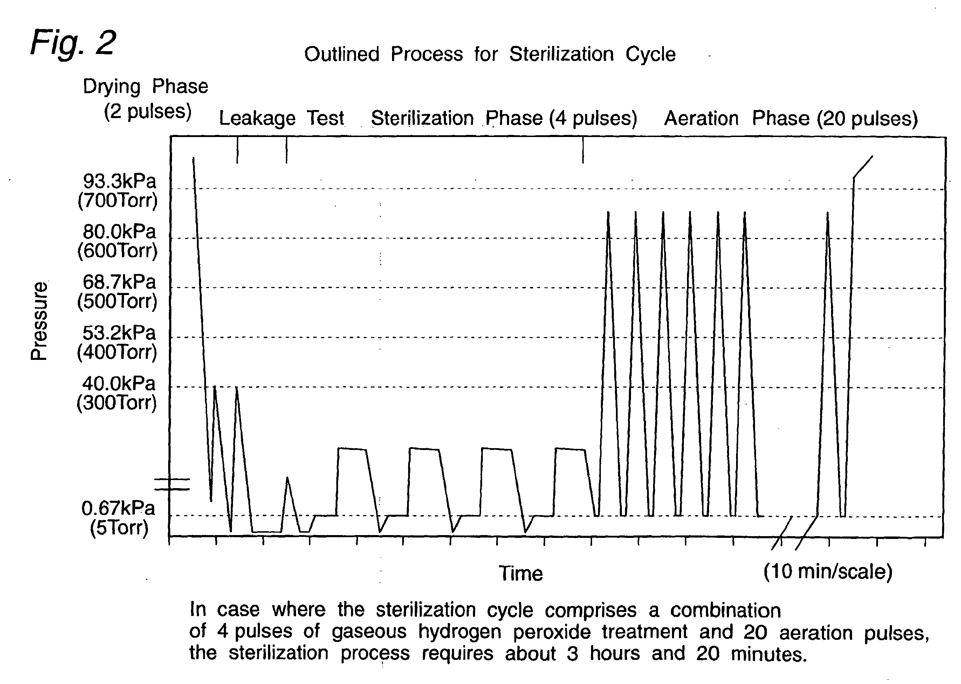

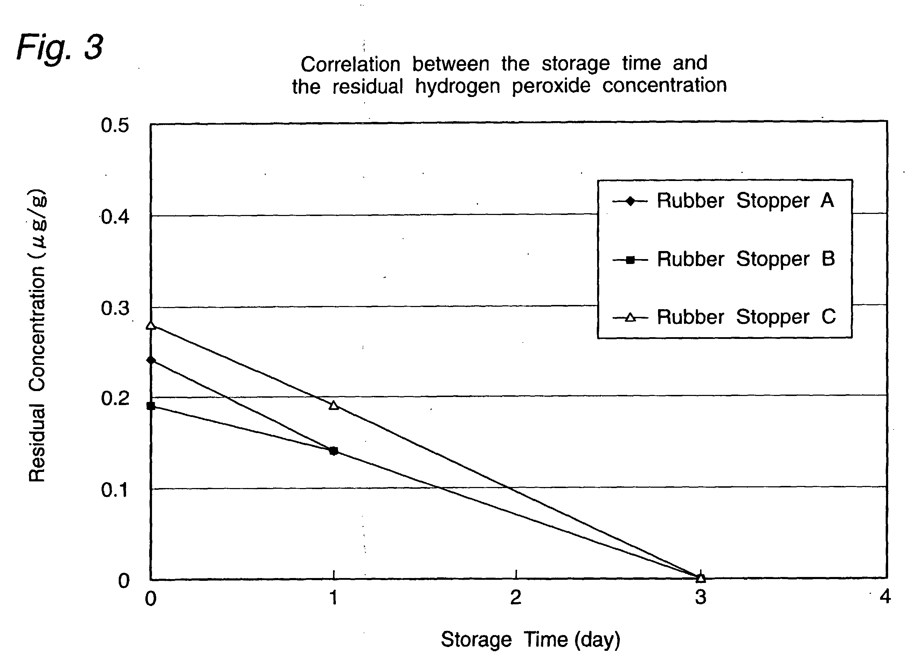

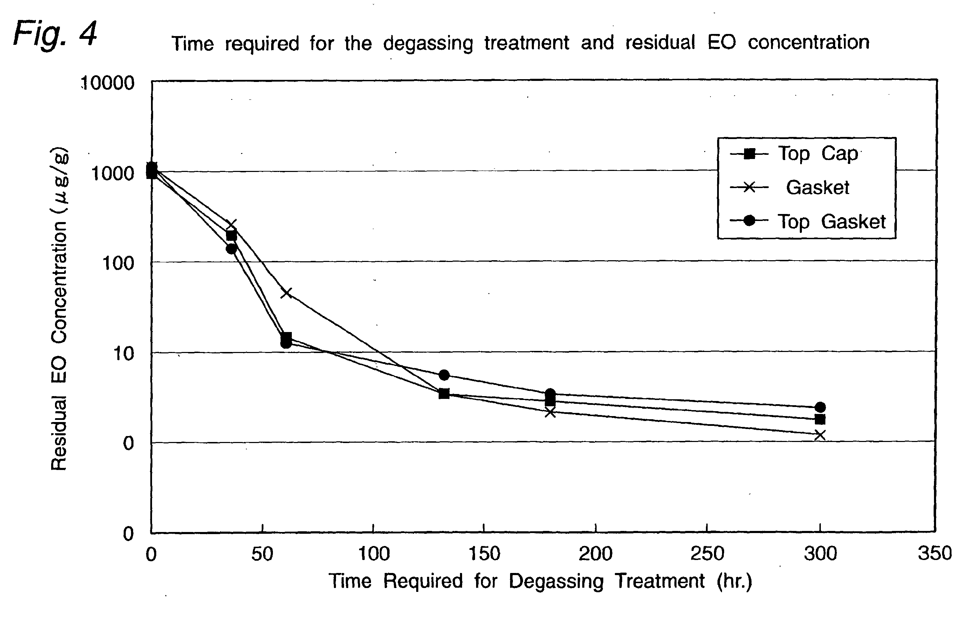

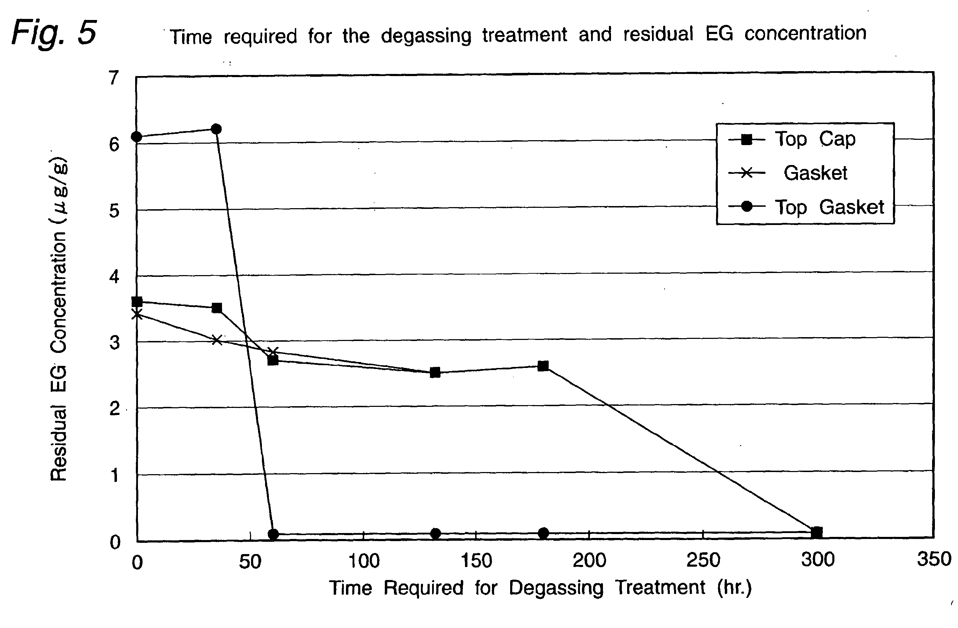

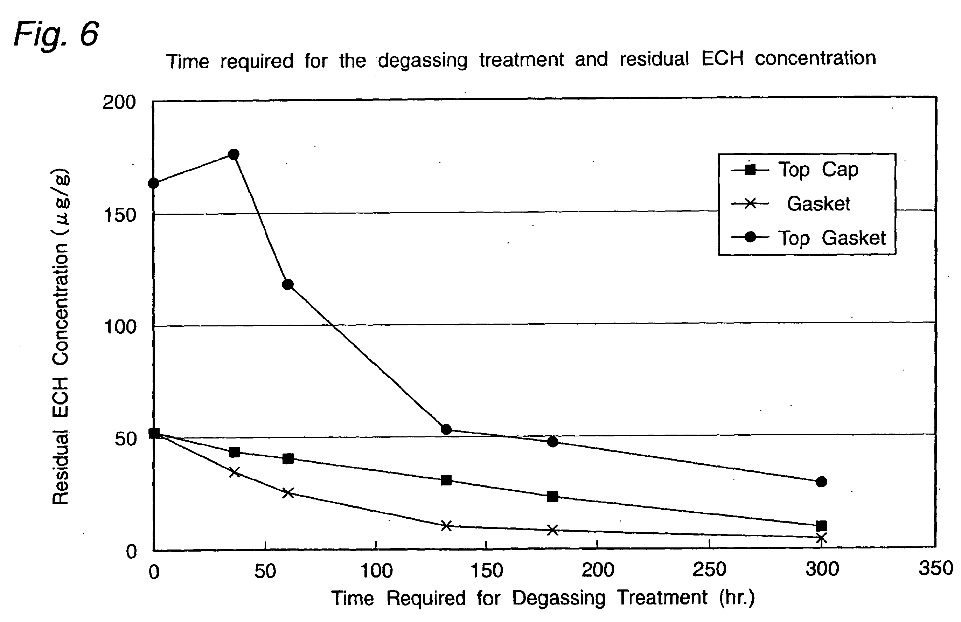

Fig. 1 is a schematic circuit diagram showing a preferred embodiment of the sterilization device according to the present invention. Fig. 2 is a chart or a flow diagram showing an example of the sterilization cycle according to the present invention, in which the time is plotted as abscissa (10 min/scale) and the internal pressure of the device is plotted as ordinate, and the pressure is expressed in terms of kPa (Torr) unit. Fig. 3 shows the correlation between the storage time after the completion of the sterilization and the concentration of the residual hydrogen peroxide, in which the storage time (day) is plotted as abscissa, while the residual hydrogen peroxide concentration (μg/g) is plotted as ordinate. Fig. 4 shows the correlation between the degassing time (h) and the residual EO concentration (μg/g) observed on a sample when using ethylene oxide gas (EOG) as a conventional sterilization agent, in which the degassing treatment time (h) is plotted as abscissa and the residual EO concentration (μg/g) is plotted as ordinate. Fig. 5 shows the correlation between the degassing time (h) and the residual EG concentration (μg/g) observed when using EOG as a conventional sterilization agent, in which the degassing treatment time (h) is plotted as abscissa and the residual EG concentration (μg/g) is plotted as ordinate. Fig. 6 shows the correlation between the degassing time (h) and the residual ECH concentration (μg/g) observed when using EOG as a conventional sterilization agent, in which the degassing treatment time (h) is plotted as abscissa and the residual ECH concentration (μg/g) is plotted as ordinate.

1 Sterilization device (whole) used in the method according to the present invention;
2 Sterilization treatment unit (sterilization chamber);
4 Gaseous hydrogen peroxide supply conduit;
6 Gaseous hydrogen peroxide supply device;

8 Switching valve fitted to the gaseous hydrogen peroxide supply conduit on the side of the sterilization chamber;

10 Switching valve fitted to the gaseous hydrogen peroxide supply conduit on the side of the gaseous hydrogen peroxide supply device;

12 Vacuum pump (aspiration means);

14 Evacuation conduit;

16 Switching valve fitted to the evacuation conduit on the upstream side of the vacuum pump;

18 Switching valve fitted to the delivery conduit on the downstream side of the vacuum pump;

20 Catalyst charged in the delivery conduit;

21 Sterilization bag (sterilization basket; not shown);

22 Separate switching valve positioned between two switching valves fitted to the gaseous hydrogen peroxide supply conduit and communicated to the air-intake opening;

23 Extended conduit for connecting the separate switching valve and the air-intake opening;

24 Air-intake opening;

26 Sterilizing filter;

28 manometer for detecting the internal pressure of the sterilization chamber;

30 Thermometer for detecting the internal temperature of the sterilization chamber.

[Best Mode for Carrying out the Invention]

<As to the Gaseous hydrogen Peroxide>

The term "gaseous hydrogen peroxide" used for carrying out the method for the production of a sealing tool by sterilization according to the present invention includes hydrogen peroxide vapor, a hydrogen peroxide-containing gas or an aerosol of hydrogen peroxide prepared by, for instance, atomization of a hydrogen peroxide solution.

In this respect, the hydrogen peroxide vapor means an oxidizing gas mainly comprising a hydrogen peroxide gas volatilized from a hydrogen peroxide aqueous solution having a concentration of not less than 30% by weight, preferably, 35% by weight and the hydrogen peroxide aqueous solution generates a hydrogen peroxide gas exhibiting even higher oxidation power as the concentration of the solution increases. In other words, the higher the content of hydrogen peroxide molecules in the vapor thus generated, the higher the efficacy of the vapor as the sterilization agent. Referring to such a sterilization action, it is understood that hydrogen peroxide molecules are decomposed on the sterilization conditions and generates at least one of the group consisting of active oxygen and hydroxide radical, the active oxygen or hydroxide radical thus generated or both of them exert(s) an oxidization action on the intended bacteria cells and the like which are to be oxidized and decomposed. For example, the "concentration of 35% by weight" is not restricted to a very precise value but usually means a concentration band having a flow with the "concentration of 35% by weight (or 35 wt%).

In addition, the hydrogen peroxide-containing gas herein used also includes an oxidizing gas entrained by an inert gas stream obtained when such an inert gas, in general, a gas inert to hydrogen peroxide such as nitrogen gas and air is passed through a hydrogen peroxide aqueous solution.

Moreover, the term "an aerosol of hydrogen peroxide prepared by atomization of a hydrogen peroxide solution" used in another embodiment of the method for the present invention means a gas-liquid mixed system obtained by converting an aqueous hydrogen peroxide solution into fine droplets, using an atomizer as an atomizing device to such an extent that the resulting aerosol is almost identical to a gas. The effect thereof as an oxidizing agent is likewise substantially identical to that observed for the gaseous hydrogen peroxide.

<As to Conditions of Sterilization Treatment>

The conditions of the treatment to be used in the present invention depends on the size of a sample to be treated, a filling rate in a sterilizing device, the state of a surface of the sample, and the concentration of gaseous hydrogen peroxide to be used and the number of sterilization pulses.

According to the experiment of the present inventor, it was observed that a survival ratio (a survival ratio of bacteria cells) is rather low when a filing rate of the sample in a sterilization bag is high, and the survival ratio tends to be increased if the filling rate is reduced (second to fifth embodiments).

As a result of investigations of a sterilization bag to be used for accommodating a sample to carry out a sterilization treatment, an outer bag for further accommodating the sterilization bag having the sample, a porous container (for example, a basket) for housing the outer bag accommodating the sterilization bag having the sample and having an opening sealed in a sterilizing device (a sterilizer) by a stacking process, and a process for mounting the outer bag in the porous container (the number of the outer bags and the presence of a dummy bag), it was observed that the survival ratio also tends to be reduced (the sterilization effect is increased) with a reduction in a rate (a volume rate) of a volume (an outer volume) of the sterilization bag to an inner volume of the porous container (sixth embodiment).

<As to Materials for Articles to be processed>

Articles (subjects to be processed) treated by the sterilization method according to the present invention are not restricted to any particular one, but favorably used herein include a variety of sealing tools since if the sterilization method for the present invention is applied thereto, the desired effects of the present invention can be ensured. As materials for these sealing tools, there may be listed, for instance, glass, ceramic wares and metals whose surface is hard and smooth, since it is very easy to achieve conspicuous effect of the present invention in case where the method is applied to these sealing tools having smooth and hard surface.

On the other hand, the results of the amount of hydrogen peroxide remaining in an article to be processed such as a sealing tool, a container, a joint, a cap or a plunger, which is formed from a resin and whose surface gives such an impression that it is apparently hard, variously vary depending on the kinds of resins used immediately after the sterilization treatment (through an aeration stage) or after several days from the treatment. In general, it has been found that the residual amount of hydrogen peroxide is substantially small if using a polyolefin resin (PO; 1-olefinic resin) such as polyethylene (PE) or polypropylene (PP) and poly(tetrafluoroethylene) (PTFE) as such a resin material and therefore, these resins can suitably be used in the present invention.

If the sealing tool is formed from a soft material such as rubber (an elastomer) or a thermoplastic elastomer (thermoplastic rubber; TPE or TPR), however, the sterilization effect is rather lower than expected. The reason of this has not yet been clearly elucidated, but it would be most probable that this is because the formation of a large number of fine unevenness on the surface (exterior).

As has been discussed above, the usual sterilization treatment with liquid hydrogen peroxide hardly kills the microorganisms penetrated into the interior of an article to be processed slightly behind the surface thereof. In this connection, it would be estimated that microorganisms may easily penetrate into the interior of a soft material such as rubber (an elastomer) or a thermoplastic elastomer (thermoplastic rubber), while hydrogen peroxide as a sterilization agent hardly penetrates into the interior thereof as compared with microorganisms and accordingly, it would be difficult that the sterilization agent is brought into contact with such microorganisms.

The foregoing rubber (or elastomer) material can roughly be divided into the conjugated diene rubber and the rubber other than the conjugated diene rubber (the non-conjugated diene rubber).

- Examples of conjugated diene rubber materials are natural rubber (NR), a variety of synthetic rubbers, blends of at least two members selected from these natural and synthetic rubber materials or copolymer rubber materials comprising repeating units of these rubber materials and other repeating units copolymerizable therewith. Specific examples of synthetic rubber materials are 1,4-cis-polyisoprene (IR) rubber obtained by 1,4-addition polymerization of isoprene, which is a repeating unit mainly constituting the natural rubber, 1,4-cis-polybutadiene rubber (BR) and isobutene- isoprene copolymer rubber (IIR; also referred to as "butyl rubber"). In this respect, halogen-substituted, in particular, chlorine-substituted derivatives of the foregoing various rubber materials have likewise widely been employed. Preferred examples thereof are chlorobutyl rubber materials.
- The non-conjugated diene rubber is a copolymer rubber material of at least two 1-olefins or a multi-component copolymer rubber material obtained by copolymerizing these monomers with a third non-conjugated diene monomer. Specific examples thereof are listed below.

The copolymer rubber material of at least two 1-olefins is, for instance, ethylene-propylene copolymer rubber (EPM), ethylene-1-butene copolymer rubber (EBM) or propylene-1-butene copolymer rubber (PBM).

The multi-component copolymer rubber (EPDM) obtained by copolymerizing at least two 1-olefins with a third non-conjugated diene is, for instance, ethylene- propylene-1,4-hexadiene copolymer rubber, ethylene-propylene-dicyclopentadien, ethylene-propylene-methylene norbornene copolymer rubber and ethylene-propylene-ethylidene norbornene copolymer rubber.

On the other hand, the thermoplastic elastomer (thermoplastic rubber) is a polymer simultaneously having characteristic properties of both the thermoplastic resin and the elastomer. More specifically, it can be formed into a variety of shapes depending on the molding processes selected and can be subjected to a vulcanization treatment (crosslinking treatment), which is usually applied to the elastomer. For this reason, the molded body obtained from the thermoplastic rubber is flexible, not so low as to the rubber material itself, but the rubber component alone is not extracted from the thermoplastic rubber material by solvent extraction thereof. This would be because the resin moiety and the elastomer moiety of the thermoplastic rubber are chemically bonded together. If it is difficult to make an elastomer material crosslinked, however, the thermoplastic rubber material also includes those obtained by kneading resins with elastomers free of such crosslinking therebetween.

In addition, if the elastomer moiety has an ability of crosslinking, but the degree of crosslinking seems to remain relatively low, however, such crosslinking is in general referred to as "partial crosslinking" or "half crosslinking".

Various kinds of thermoplastic elastomers (TPE; or TPR: thermoplastic rubber materials) can be used in the sterilization method for the present invention as the material for the article to be processed. However, suitably used herein are, from the practical standpoint, partially crosslinked (or half-crosslinked) olefinic thermoplastic elastomers prepared by kneading, at a high temperature, a composition (or a mixture) containing thermoplastic polyolefin resins (PO) and multi-component copolymer rubber (EPDM) materials or further kneading the foregoing composition at a vulcanization temperature in the coexistence of a radical initiator (a crosslinking agent or a vulcanizing agent) such as a peroxide.

Examples of these olefinic thermoplastic elastomers (TPO) include those comprising, as ingredients, a combination of a polyolefin resin (PO) such as a polyethylene resin (PE) with an ethylene-propylene-ethylidene norbornene copolymer rubber (E-P- ENB) material; a combination of a polyethylene resin (PE) with an ethylene-propylene- methylene norbornene copolymer rubber (E-P-MNB) material; and a combination of a polyethylene resin (PE) with an ethylene-propylene-1,4-hexadiene copolymer rubber (E-P-HXD) material; as well as the foregoing various combinations in which the polyethylene resin (PE) component is replaced with at least one member selected from the group consisting of polypropylene resins (PP) and propylene-1-butene copolymer resins.

The sterilization method according to the present invention, which makes use of gaseous hydrogen peroxide, permits the substantially effective sterilization of even the foregoing microorganisms penetrated into the interior of the article to be processed, whose extermination has conventionally been considered to be quite difficult.

<Kinds of Articles to be Processed>

Specific examples of the articles to be processed include wide variety of articles such as rubber stoppers (rubber gaskets), rubber caps, rubber gaskets, gaskets (also referred to as "plunger rubber") for pistons (or plungers) inserted into injection cylinders (syringes), tools for preventing liquid leakage such as rubber boots and elastic rings for bushing, sheath, sleeves and for fitting (interposition) joints, for instance, O-rings (O-rings and rings having an approximately circular cross section). The foregoing "rubber stoppers" mainly includes gaskets for syringes (injection cylinders). In this connection, the articles to be processed according to the process of the present invention may likewise include tools of glass, metals, ceramics (including ceramic wares) and plastics as well as the sealing tool. In case where these tools have porous inner surfaces (porous wall surfaces), however, the walls thereof, which come in contact with the sterilization agent, are preferably treated, in advance, so that they can block any penetration of the sterilization agent. This is because, if the walls are not pre-treated, the removal of the residue after the sterilization often requires a great deal of labor. In order to prevent hydrogen peroxide from remaining after the treatment, particularly, it is important that inner wall surfaces (to be treated) of the tools are not hydrophilic.

The term "rubber" or "rubbery" frequently used in the present invention means isobutene-isoprene copolymer rubber (abbreviated as "IIR"; common name: "butyl rubber"). This copolymer rubber (IIR) is excellent, in particular, in the gas-barrier ability and accordingly, it has widely been used in the field of goods for medical use. However, silicone rubber (SR) and fluororubber may be used in special cases since the former is excellent in self-lubricating properties and has high temperature stability and chemical stability (resistance to chemicals) as compared with the butyl rubber (IIR) and the latter surpasses the quality of the silicone rubber. The "butyl rubber" used in the present invention also includes those commonly referred to as "butyl rubber" (this is in fact chlorobutyl rubber or butyl rubber obtained by at least partially substituting a large number of hydrogen atoms bonded to the IIR molecular chain with chlorine atoms).

Preferred embodiments according to the present invention will hereunder be described in more detail with reference to the accompanying drawings and Tables given below. However, the present invention is not limited to the scope of these specific embodiments at all.

[Description of the Invention Based on the Drawings]

The present invention will hereunder be described more specifically with reference to the drawings attached hereto. However, the present invention is by no means limited by the following description and the accompanying drawings.

<Sterilization (Treatment) Unit>

A large number of rubbery sealing tools as the articles to be processed are in general accommodated in the body of a sterilization chamber (another name: "sterilization unit" : sterilizer or sterilizer device) 2. In case where the rubbery sealing tool is a stopper or plug having a tubular or columnar shape, it is sufficient that the sterilization chamber (sterilizer) 2 is a hollow body having an inner volume generally ranging from 50 to 30000 L (0.05 to 30 m³) and preferably 1000 to 150000L (1 to 150 m$^3$).

Gaseous hydrogen peroxide is fed to the sterilization chamber 2, through a conduit 4 for the introduction of the gaseous hydrogen peroxide (hereunder abbreviated as "introduction conduit") fitted thereto on the upstream side. This gaseous hydrogen peroxide sterilizes the articles to be processed in the chamber and after the elapse of a desired mean retention time, it is discharged through a suction conduit (discharge conduit) 14 fitted to the sterilization chamber on its downstream side.

In the interior of the sterilization chamber 2, rubber stoppers (not shown; a tubular shape having the foregoing specifications; total number: 36000) as the articles to be processed are accommodated in sterilization bags (not shown; each containing about 500 articles) and every 50 sterilization bags are, in order, arranged within the sterilization chamber 2, so that they come close to one another and extend from the upstream side (top plate side) towards the downstream side (bottom plate side).

It is preferred to control the degree of the opening or vacant space (space ratio), which is defined to be a rate of the space within the sterilization chamber 2 (inner volume: about 8.6 m$^3$) other than that occupied by the articles to be processed and, in case where means for putting or suspending the articles is used, occupied by both the articles and the means, in general ranges from 10 to 85% and preferably 50 to 70%.

In the present invention, it is preferred to pass gaseous hydrogen peroxide having a hydrogen peroxide concentration ranging from 0.5 to 4.0 mg/L (Liter), preferably 1.5 to 2.5 mg/L through the sterilization chamber 2 at an average flow rate ranging from 1000 to 10000 L/min and preferably 3000 to 5000 L/min. The sterilization chamber 2 is appropriately maintained at a mean internal temperature ranging from 15 to 55°C, preferably 20 to 40°C and at an average pressure ranging from 0.002 to 0.04 atm, preferably 0.005 to 0.015 atm.

If the sterilization treatment is carried out in this sterilization chamber 2 under the foregoing treating conditions for an average treating time ranging from 30 to 240 min, preferably 45 to 120 min, the viable count of bacterial cells: Bacillus Stearothermophilus ATCC 12980 remaining on the surface of the rubber stopper can be reduced to SAL = 10EXP(-6) (= 10$^{-6}$).

<Volume Rate in Sterilization Unit (Sterilizer; Sterilization Chamber>

It is preferable that a sterilization bag should not be exactly mounted in a sterilization device but be mounted in a state of accommodation in an outer bag. The outer bag is further mounted in a porous container and a gaseous sterilization agent penetrates from an upper opening of the outer bag through a bottom and a side wall of the container and penetrates into the sterilization bag from a bottom surface in contact with the outside of the sterilization bag. In the sterilization bag, moreover, the sterilization agent penetrates into the bag from a bottom portion so that the sterilization agent gas can uniformly permeate into the bag easily. More specifically, the sterilization agent can fully come in contact with the surface of a sample accommodated in the sterilization bag. A box-shaped container referred to as a basket is usually used as a porous container conveniently. The box-shaped container has an advantage that it can be stacked in multistage and the limited floor area can be utilized very effectively.

The sterilization bag (inner bag) usually used is a polyolefin synthetic paper, for example, a gazette type bag formed of a polyethylene synthetic paper or a flat bag formed of a polyethylene synthetic paper, and a layer thereof is constituted by a three-layer laminated film in which a polyethylene (PE) layer and a polypropylene (PP) layer are laminated on a base layer [trade name : Tyveck 1073B (produced by Du Pont Co., Ltd.)]. The bag has three kinds of lengths and has a normal outer dimension of a width 380 mm X a thickness (upward 80 mm + downward 80 mm) X a length 350 mm, 500 mm or 800 mm. It is sufficient that the outer dimension of an outer bag formed of polyethylene having a low density further accommodating a sterilization bag having a sample to be sterilized (the sterilization bag containing the sample) can fully accommodate the sterilization bag containing the sample.

In other words, the outer dimension of the outer bag is properly selected according to the standards of the sterilization bag. For reference, an outer bag which is used most often has an outer dimension of a length 900 mm X a width 750 mm and has a mean thickness of 0.04 mm. The outer bag to be used most often is a flat polyethylene bag. As a matter of course, it can be approved that the outer dimension of the outer bag has a tolerance of approximately ±10%.

It is premised that the basket to be usually used as a porous container for mounting the outer bag having the example can stand the weight of the mounted article and a self-weight and has such a strength as to stand the loads of the basket stacked thereon and the mounted article when it is piled up. In addition, it is premised that such a reduction in the strength as to make troubles is not generated even if the basket is exposed to a hydrogen peroxide gas (gaseous hydrogen peroxide) for a long time. Accordingly, it is preferable that the material of the basket should be a synthetic resin, particularly, a synthetic resin rarely containing an unsaturated linkage, a fiber-reinforcedmaterial or a metal. While a mineral fiber such as a glass fiber, a carbon fiber or a metallic fiber is suitable for the fiber for reinforcement, a fiber made from a synthetic resin can also be used for the reinforcement depending on circumstances. In this case, the synthetic resin fiber for the reinforcement may be made from a thermosetting resin or a thermoplastic resin and it is desirable that a crystal melting point or a softening point of the latter resin should be much higher than that of a base resin (matrix resin), for example, should be higher by 10°C or more, preferably 20°C or more.

The basket used in the present invention is classified into three types for each content, and a basket 1 (inner volume : 45 L) , a basket 2 (inner volume : 62 L) and a basket 3 (inner volume : 161 L) are employed.

Two outer filled bags are mounted in parallel in the baskets. An experiment is carried out in a sixth embodiment and the result thereof is shown in Table 8.

It is defined that a volume rate (%) = an outer volume (an occupied volume) of a filled outer bag / an inner volume of a basket is set. The interpretation of data in the Table 8 based on the definition of the volume rate is as follows:

- A survival ratio could not be always set to 0/10 for the number of times of sterilization of 1 to 5 with a volume rate to be a maximum set value of 67 %.
- However, the survival ratio could be set to 0/10 for the number of times of sterilization of 1 to 5 with a volume rate of 48 % and 16 %.

<Sample Filling Rate in Sterilization Bag>

According to the investigation of the present inventor, a ratio (a filling rate) of the sample to be sterilized in the sterilization bag is shown in Tables 4 to 7. The following is apparent from the Tables 4 to 7:

In the case in which a gasket for a normal type syringe (VF1 : outer diameter 7 mm X overall height 6 mm ; VF3 : outer diameter 9 mm X overall height 8 mm ; VF5 : outer diameter 12 mm X overall height 10 mm) is to be filled in the sterilization bag, it is desirable that a filling rate should be set to 45 % or more, preferably, 50 % or more in order to always cause a survival ratio to reach 0/10 in the sterilization treatment in which the sterilization condition is set to an injection amount of hydrogen peroxide to be a sterilization agent 70 (g/pulse) X 3 pulses + aeration 20 pulses.

On the other hand, in the case in which a gasket for a large-sized syringe (outer diameter 42 mm X overall height 36 mm ; a gasket of a plunger, a piston portion of the plunger) is filled in the sterilization bag and the sterilization condition is set to an injection amount of hydrogen peroxide 70 (g/pulse) X 3 pulses + aeration 20 pulses to carry out the sterilization treatment, it is apparent from the Table 7 that the survival ratio can always reach 0/10 with a filling rate of 73 %, 53 % or 26 %.

<Final Result>

The surface and the surface layer portion in a sterilized rubber gasket thus obtained can be sterilized up to SAL = 10 EXP (-6) in bacteria cells having a name of "Bacillus Stearothermophilus ATCC 12980" to be the indicator bacteria of the hydrogen peroxide sterilization.

<Conditions and Procedures of Sterilization Treatment>

We will hereunder describe in detail the conditions for the sterilization treatment used in the present invention such as the concentration of gaseous hydrogen peroxide (including hydrogen peroxide gas and hydrogen peroxide vapor) or the content of the "hydrogen peroxide", the flow rate of the gaseous hydrogen peroxide, the treating temperature and pressure, the shape and the placed condition of the treating unit, the placed condition of the article to be processed (such as placed position, placed density and the direction of place), the treating time and the degree of agitation in the treating unit.

(1) Gaseous Hydrogen Peroxide

(1.1) Concentration: The content of hydrogen peroxide ($H_2O_2$) in general ranges from 2.9 to 14.7 mol/L, preferably 8.8 to 10.3 mol/L; or 100 to 500 g/L and preferably 300 to 350 g/L.

(1.2) Flow Rate: This is in general controlled to the range of 500 to 5000 L/h (N.T.P.) and preferably 1400 to 2400 L/h (N.T.P.).

(1.3) Treating Temperature and Pressure: They are in general adjusted so as to fall within the range of 15 to 55°C and 0.2 to 50 Pa, preferably 20 to 40°C and 0.5 to 30 Pa, respectively.

(1.4) Shape and Placed Condition of Sterilization Chamber 2: A fixed hollow rectangular prism (chamber, unit or container) or a rotatable and/or rollable hollow cylinder (drum); in this connection, spherical bodies (such as steel balls) or other means for accelerating the mutual contact may be coexistent in the drum.

(1.5) Shape and Placed Condition of Article to be Processed: The article to be processed may have a variety of shapes such as a columnar shape, a truncated cone-like shape, a tubular shape, a circular disk-like shape, a flanged columnar shape and a hollow single-ended cap. They may be treated in a box-like or bath-shaped sterilization chamber, while they are randomly accommodated in sterilization bags and the resulting bulk materials are placed or suspended in the chamber; or in a rotatable and/or rollable drum, while they are accommodated in sterilization bags, by the action of the rotation and/or rolling of the sterilization chamber in an irregularly oriented (randomly arranged) condition.

Manner of Arrangement of Articles to be Processed: There has widely been adopted a method in which a large number (several thousands of articles/sterilization bag) of articles (for instance, "syringe gasket"; another name: "syringe piston") to be processed are randomly (irregularly) accommodated in bags. In case where the articles are treated in a rotatable and/or rollable drum according to an alternative method, this irregular accommodation thereof in sterilization bags is likewise adopted in most of cases and it is sufficient for these methods to accomplish the usual purpose and to thus sufficiently achieve a desired effect.

(1.6) Manner of Contact within Treating Chamber: As an embodiment usually adopted for the sterilization of normal-sized syringe gaskets, for example, VF1 (outer diameter 7.0 mm X overall height 6 mm), VF3 (outer diameter of 9.0 mm X overall height 8 mm) and VF5 (outer diameter 12.0 mm X overall height 10 mm), about 2000, 10000 or 4000 syringe gaskets are in general accommodated in a sterilization bag and about 50 bags filled with the syringe gaskets are subjected to the sterilization treatment in one lot, in a batchwise manner.

<Preferred Embodiments>

Fig.1 is a circuit diagram showing the outline of a sterilization device according to one embodiment of the present invention. In Fig. 1, the reference numeral 2 represents a "sterilization chamber " for accommodating articles to be processed within the sterilization device and a device 6 for the supply of gaseous hydrogen peroxide is connected to the sterilization chamber 2 through a conduit 4 for the supply of gaseous hydrogen peroxide. Switching valves 8 and 10 are fitted to the gaseous hydrogen peroxide supply conduit 4 on the side of the sterilization chamber 2 and on the side of the gaseous hydrogen peroxide supply device 6, respectively.

In addition, a vacuum pump 12 for evacuating the gas present in this sterilization chamber 2 is connected to the sterilization chamber 2 through an evacuation (or aspiration) conduit 14 and a switching valve 16. The gas aspirated by the vacuum pump 12 is externally discharged from the device after coming in contact with a catalyst 20 positioned in an exhaust conduit 18 arranged on the downstream side. Moreover, an air-intake opening 24 is connected to the chamber 2 through a separate switching valve 22 and extension conduit 23 positioned between the foregoing two switching valves 8 and 10, which are arranged on the gaseous hydrogen peroxide supply conduit 4. The air is introduced into the sterilization chamber 2 through the air-intake opening 24 and a sterilization filter 26, which is connected to the gaseous hydrogen peroxide supply conduit 4. The sterilization chamber 2 is equipped with a pressure indicator 28 and a thermometer 30 for detecting the internal pressure and temperature of the chamber 2, respectively.

<<Sterilization Pulse and Aeration Pulse>>

We will hereunder explain in more detail the sterilization steps using the sterilization device having the structure described above. First of all, articles to be processed (articles to be sterilized; not shown) are introduced into the sterilization chamber 2 according to a variety of introduction methods and then the sterilization chamber 2 is sealed by closing the door thereof. At this stage, the internal pressure of the chamber 2 is approximately equal to the atmospheric pressure.

Then, the vacuum pump 12 is put into operation and the switching valve 16 fitted to the aspiration conduit 14 of the vacuum pump 12 is opened to aspirate or evacuate the sterilization chamber 2 and to thus establish a high vacuum within the chamber. The degree of the high vacuum commonly used in the sterilization pulse according to the present invention is initially about 0.13 kPa (1 Torr). Then after a desired degree of vacuum is established within the chamber, the switching valve 16 on the vacuum pump 12 side is closed, followed by opening the switching valves 8 and 10 arranged on the gaseous hydrogen peroxide supply conduit 4 to thus introduce the gaseous hydrogen peroxide accommodated in the gaseous hydrogen peroxide supply device 6 into the sterilization chamber 2 through these valves and to uniformly distribute the gaseous hydrogen peroxide within the chamber 2. The concentration (internal gas concentration) of the gaseous hydrogen peroxide usually used at this stage is about 1500 ppm.

It is important to supply gaseous hydrogen peroxide in a desired amount. More specifically, if the amount of the gaseous hydrogen peroxide supplied is too great, it is difficult to maintain the gaseous hydrogen peroxide in the sterilization chamber 2 in a gaseous state. However, the internal air pressure of the sterilization chamber 2 is extremely low as a result of the evacuation by the vacuum pump 12 and accordingly, the partial pressure of the gaseous hydrogen peroxide in the chamber is in fact increased.

<< Parameters for Operating Sterilization Cycle>>

According to an embodiment described in the following Table 1, one sterilization pulse in the sterilization step of the present invention is maintained at a high vacuum state in general for 4 minutes, then the switching valve 22 connected with the air-intake opening 24 is opened to introduce sterilized air, which has passed through the sterilization filter 26, into the sterilization chamber 2 and the chamber is maintained at this state usually for 6 minutes so that the gaseous hydrogen peroxide can enter into and/or penetrate into the whole portions of the articles to be processed. As a result, the internal pressure of the sterilization chamber 2 rises up to the usual pressure of 21.98 kPa (165 Torr) slightly higher than the vacuum state. Thereafter, the foregoing sterilization pulse is in general repeated over desired times (desired number of pulses). It is in general sufficient to repeat this sterilization pulse twice, but the pulse is preferably repeated 4 times.

Subsequent to this sterilization pulse, an aeration pulse is carried out. This aeration pulse comprises the steps of introducing sterile air into the sterilization chamber 2 to establish a desired pressure therein and maintaining the chamber 2 at that condition for a predetermined period of time to thus remove the residual hydrogen peroxide from the chamber 2. To achieve the purpose of the aeration pulse, it is sufficient to set the time required for this aeration pulse to about 7 minutes/pulse.

This aeration pulse is usually repeated over desired times. The repeated number of the aeration pulses is in general set at about 20. A series of these pulses permit the effective execution of a desired sterilization treatment. The parameters (conditions for operations) involved in the sterilization cycle of the present invention will be illustrated in the following Table 1.

Table 1:

| Parameters for Sterilization Cycle | | |
|---|---|---|
| Sterilization Step | Subject to be Established | Value Established |
| Dry Phase | Number of 1st Drying Pulse | 1 Pulse |
| | Established Pressure in the 1st Drying | 1.33 kPa (10 |
| | Pulse | Torr) |
| | Number of 2nd Drying Pulse | 1 Pulse |
| | Established Pressure in the 2nd Drying | 0.13 kPa (1 Torr) |
| | Pulse | 39.99kPa (300 |
| | Established Pressure at the Pressure | Torr) |
| | Restoration | |
| Leakage Test | Established Pressure at Pressure | 0.13kPa (1 Torr) |
| | Reduction | 5 minutes |
| | Retention Time in Leakage Test | 6.66kPa (50 |
| | Established Pressure at the Pressure | Torr) |
| | Restoration | |

Table 1: (continued)

| Sterilization Step | Subject to be Established | Value Established |
|---|---|---|
| Parameters for Sterilization Cycle | | |
| Sterilization Phase | Number of Sterilization Pulse | 4 Pulses |
| | Established Pressure at Pressure | 0.13kPa (1 Torr) |
| | Reduction | 40 g (35% Aq. |
| | Amt. Of Sterilization agent per Pulse | Soln.) |
| | Retention Time Immediately After | 4 minutes |
| | Injection of the Sterilization Agent | |
| | Established Pressure at the Pressure | 21.98kPa (165 |
| | Restoration | Torr) |
| | Retention Time After Pressure | 6 minutes |
| | Restoration | FAST |
| | Establishment of Pressure Restoration | |
| | Rate | |
| Aeration Phase | Number of Pulses | 20 pulses |
| | Established Pressure at Pressure | 0.67kPa (5 Torr) |
| | Reduction | 86.66kPa (650 |
| | Established Pressure at the Pressure | Torr) |
| | Restoration | |
| Prior Conditions for the Data listed in Table 1: As a sterilization chamber (sterilization kettle), there was used a chamber having an inner volume of 8.6 m$^3$, and the sterilization comprises 4 sterilization pulses and 20 aeration pulses. | | |

<<Arrangement of These Two Kinds of Pulses>>

The foregoing steps may roughly be divided into the "sterilization pulse" and the "aeration pulse" subsequent thereto and the repetition of each of these two kinds of pulses over a plurality of times would make the effectiveness of the sterilization treatment higher. To effectively carry out the sterilization treatment according to the present invention, it is effective to combine, in order, 2 to 20 times, preferably 3 to 10 times of the sterilization pulses and 5 to 50 times, preferably 10 to 30 times of the aeration pulses. The expression "if the number of sterilization pulses is set to 3 (or 4)" in the component of the present invention is not restricted but is a prior condition (a criterion) for specifying the filling rate of bacteria cells to be sterilized which are accommodated in the sterilization bag.

After completion of the sterilization of articles to be processed according to the foregoing steps, the aeration pulses are initiated, in which the gaseous hydrogen peroxide is removed from the sterilization chamber 2.

The sterilization chamber 2 is again evacuated by the action of the vacuum pump 12 to thus establish a vacuum (a reduced pressure) of a desired pressure within the chamber 2. After the establishment of a desired vacuum, the switching valve 16 on the side of the vacuum pump 12 is closed, while the switching valve 22 to the air-intake opening 24 and the switching valve 8 of the gaseous hydrogen peroxide supply conduit 4 on the side of the sterilization chamber 2 are opened to thus introduce sterilized air into the sterilization chamber 2 and to restore the internal pressure of the chamber 2 to a pressure slightly lower than the atmospheric pressure.

At this stage wherein the sterilization and aeration pulses according to the present invention are completed, the gaseous hydrogen peroxide can be decomposed and removed within a short period of time according to a decomposition method, which makes use of a commonly used catalyst. After the removal of the gaseous hydrogen peroxide present in the sterilization chamber 2, the sterilization chamber 2 can be opened to remove the sterilized articles. This sterilization process never suffers from a problem of environmental pollution since the decomposition products obtained after the sterilization are oxygen and water.

According to the sterilization device of the present invention, the amount of the gaseous hydrogen peroxide remaining in the sterilization chamber 2 can be reduced to a level of not more than 1 ppm because of the contribution of the aeration performed before the withdrawal of the articles after the sterilization treatment.

The sterilization method according to the present invention is suitably applied not only to the foregoing articles of resins such as syringes for injection and containers for ophthalmic medicaments, but also to a variety of articles, in particular, those having poor heat resistance and to high speed sterilization of articles having complicated shapes.

The gaseous hydrogen peroxide used as the sterilization gas in the present invention is in general supplied to the sterilization chamber 2 through the top board (or top panel) thereof, penetrates into the sterilization bag placed in the sterilization chamber 2 through a large number of through holes (meshes, stitches), comes in contact with the surface of the articles to be processed, thus a part thereof is consumed, but the hydrogen peroxide is uniformly distributed (spreaded) throughout the entire sterilization bags, since the sterilization is carried out under a high vacuum.

The foregoing sterilization bag can be prepared by, for instance, putting porous films formed from a soft resin or the like on top of each other and then fusion-bonding the peripheral edges thereof; or by knitting a fine band-like or fibrous material of, for instance, a soft resin or converting the material into (air-permeable) sheets (or films) having a large number of through holes in a span-bonding process, then putting them on top of one another and fusion-bonding the peripheral edges thereof.

<<As to the Method for Determining (Quantifying) the Concentration of the Residual Sterilization Gas (Gaseous Hydrogen Peroxide)>>

The concentration of the residual hydrogen peroxide gas was determined according to the ammonium thiocyanate method. This ammonium thiocyanate method comprises the following operations:

When a variety of articles sterilized by means of the device hereunder are container in shape, they can be directly filled with purified water prior to the determination of the concentration of a sterilizing agent. They have been sterilized by means of the foregoing high vacuum hydrogen peroxide-sterilization device (trade name VHP DV1000 available from Steris Company; hereunder referred to as "VHP Device").

While in case of, for instance, caps for ophthalmic medicaments, nozzles, rubber stoppers or sterilization bags, these samples are accommodated in a conical flask (inner volume: 200 ml; a flask with a ground-in stopper), 100 ml·of purified water is added to the flask and these samples are immersed in the purified water maintained at 20°C (or room temperature) for 24 hours.

The resulting extract (5 ml each) was measured and poured into a test tube (with a ground-in stopper), 1 ml of a 1% aqueous solution of iron sulfate is added thereto, followed by sufficient stirring, addition of 5 ml of an aqueous ammonium thiocyanate solution (1.2 mol/L; M/L) for coloration and additionally stirring sufficiently.

This colored sample solution was measured for the absorbance at a wavelength of 480 nm using a spectrophotometer.

Similarly, a hydrogen peroxide reference solution (concentration: 5 ppm), purified water and an article free of any sterilization treatment were measured for the absorbance at the same wavelength.

The hydrogen peroxide concentration of each sample solution can be calculated based on the following formula (1):

$$C = (Abs - Abs0) \times N \times F \tag{1}$$

Wherein each symbol has the following meaning:

C: The concentration (ppm) of hydrogen peroxide to be determined;
Abs : The absorbance of each sample solution as determined using VHP Device after the sterilization treatment;
Abs0: The absorbance of each sample solution as determined using VHP Device prior to the sterilization treatment;
AbsH: The absorbance of the hydrogen peroxide reference solution;
AbsW: The absorbance of purified water;
N: The dilution factor of the sample solution;
S: The concentration (ppm) of the hydrogen peroxide reference solution; and
F: A coefficient calculated using the hydrogen peroxide reference solution according to the following formula (2):

$$F = S/(AbsH - AbsW) \tag{2}$$

<<As to the Precision of the Ammonium Thiocyanate (Quantification) Method>>

The ammonium thiocyanate method comprises the steps of converting bivalent iron ions (ferrous ions) added to an acidic aqueous solution as an agent into trivalent iron ions (ferric ions) while making use of the oxidation action of hydrogen peroxide in the acidic solution, forming an iron thiocyanate complex colored red through the reaction of the trivalent iron ions with thiocyanate ions and determining the absorbance of the complex to calculate the concentration of the hydrogen peroxide present in the solution. However, the degree of coloration is apt to vary with time and therefore, the determination of the coloration should be completed within one hour.

The calibration curve (represented by the following formula (3)) required in this determination was prepared on the basis of the absorbance values (X) as a function of the concentration (Y) of the hydrogen peroxide reference solution.

$$\bullet \text{ Calibration Curve: } Y = 3.480X - 0.142 \qquad (3)$$

Separately, the calibration curve was inspected for the ability of quantification and the results thus obtained were statistically analyzed to obtain the correlation coefficient ® and it was found to be 0.999958. This clearly indicates that this calibration curve has a considerably high correlation (reliability). It was confirmed that the foregoing determination method (the calibration curve) permits the measurement of the concentration of an aqueous hydrogen peroxide solution within the range of 0 to 14 ppm.

<<As to the Detection Limit of the Ammonium Thiocyanate Method>>

To confirm or determine the lowest possible concentration of hydrogen peroxide capable of being detected by the ammonium thiocyanate method (detection limit of the method), hydrogenperoxide solutions having three concentrations: 5.00 ppm, 0.5 ppm and 0.05 ppm (6 samples each) were prepared, the order of measurement thereof was determined using a table of random digits as specified in JIS Z9031, and each sample solution was measured for the absorbance and coefficient (ppm/Abs) at a wavelength of 480 nm to thus investigate the relation between them. As a result, the measurement error ($\pm 2\sigma$) for each measurement thus calculated was found to be $\pm 0.053$ ppm for the concentration of 5.00 ppm, $\pm 0.007$ ppm for the concentration of 0.50 ppm and $\pm 0.009$ ppm for the concentration of 0.05 ppm.

<<Tools and Materials or the like used in the Measurement>>

1. Principal Installation

1.1 Hydrogen Peroxide Vapor-Generation Device

- Type (trade name): VHP DV1000 (available from Steris Company);
- Amt. Of Injected hydrogen Peroxide: about 4 to 400 g/pulse.

1.2 Sterilization Chamber (Sterilization Unit)

- Internal Volume: 8.6 $m^3$;
- Material: SUS304
- Vacuum Pump Unit;

  Type: TRM1253+TRA1002 (available from Unozawagumi Tekkosho Co., Ltd.);

- Capacity: 760 to 1 Torr; accessible time: within 5 minutes. 1.3 Catalyst for Decomposing Hydrogen Peroxide Gas
- Specifications: Platinum-containing catalyst; Corresponding to discharge concentration of not more than 1 ppm.

1.4 Filters

- Pre-filter: Made of PP; corresponding to 10 µm abs; one filter (three elements);
- Sterilized Filter: Made of PTFE; corresponding to 0.22µm abs; two filters (three elements each)

2. Principal Materials, Fixtures, Room Environment

2.1 Validator

- Type: X1310CE (main body); LTR-140 (Dry Well Calibrator for low temperature);
- M2801/IRTD-400 (high precision thermometer)
- Manufacturer: KAYE; portable validator

2.2 Device for Measuring Gas Concentration in Sterilization Chamber (used after the completion of the sterilization)

- Manufacturer: Dreager Company;
- Dreager Accuro 2000 Gas Detection Pump;
- Dreager Gas Detector Tube (hydrogen peroxide 0.1/a)

2.3 BI Used

- Standard: $2.5 \times 10^6$ (2.5 EXP (6)) per Carrier;
- Indicator Microorganism: Apex Laboratories Co., Ltd.; Bacillus Stearothermophilus ATCC 12980.

2.4 Environment in Sterilization Chamber

- Measured Value During Automated Operation: 1,733 cells/ft$^3$ (Class 10,000).

3. Outline of Sterilization Cycle

The hydrogen peroxide vapor-generation device (VHP DV1000) has been developed for the sterilization of a freeze dryer and the sterilization cycle required for the generation of hydrogen peroxide vapor is integrated (programmed) therein. The specifications thereof will be detailed below:

3.1 Drying Step (For further details, one can refer to Table 1 and Fig. 2)

In the first drying pulse, the pressure is reduced from ordinary pressure to 1.33 kPa (10 Torr) within one minute and then the pressure is restored up to 39.9 kPa (300 Torr) within one minute. In the second drying pulse, it is reduced to 0.13 kPa (1 Torr) within one minute and it is again restored up to 39.9 kPa (300 Torr) within one minute.

3.2 Leakage Test (For further details, one can refer to Table 1 and Fig. 2)

The internal pressure of the sterilization chamber is reduced from the condition after the completion of the drying step, at which the pressure has been restored up to 39.9 kPa (300 Torr) to 0.133 kPa (1 Torr) within one minute, followed by maintaining that condition for 5 minutes to confirm if any "pressure change" is observed or not and then the pressure is restored up to 6.66 kPa (50 Torr) within one minute. 3.3 Sterilization Step (For further details, one can refer to Table 1 and Fig. 2)
After reducing the internal pressure of the sterilization chamber from 6.66 kPa (50 Torr) at the completion of the leakage test to 0.13 kPa (1 Torr) within one minute, a predetermined amount (40 to 50 g) of an aqueous hydrogen peroxide solution (concentration: about 35% by weight) is poured into the chamber, followed by maintaining the condition wherein the internal gas concentration is about 1500 ppm for about 4 minutes, restoration of the pressure to 21.98 kPa (165 Torr) by the injection of clean air into the chamber and maintaining the interior of the chamber at this pressure for 6 minutes.

The foregoing operations extending "from the establishment of a high vacuum to the internal pressure-maintenance by the injection of clean air" are defined to be one pulse and the steps for the injection of sterilizing gas and clean air are repeated over the required pulse number. The time required for the sterilization step is in general about 16 min/pulse.

3.4 Aeration Step (For further details, one can refer to Table 1 and Fig. 2)

The internal pressure of the sterilization chamber is restored from 0.67 kPa (5 Torr) observed at the completion of the sterilization step to 86.66 kPa (650 Torr) by the injection of clean air in the chamber 2 within one minute. The foregoing operations extending from the establishment of a high vacuum to the restoration of the internal pressure to about ordinary pressure is defined to be one aeration pulse and this aeration step is repeated over a desired pulse number. The time required for the aeration pulse is about 7 min/pulse.

Example 1

Three kinds of large-sized rubber stoppers for vials (they are made of chlorinated butyl rubber; comprise a cone-like head having an obtuse vertical angle and a cylindrical barrel subsequent thereto and the cylindrical barrel has a size of 42 mm (diameter)×15 mm (height) and the overall height of the stopper is 36 mm) were referred to as a rubber stopper A, a rubber stopper B and a rubber stopper C, respectively.

Each kind of rubber stoppers (150 stoppers each) were accommodated in a sterilization basket, followed by subjecting them to a sterilization step comprising 6 pulses of sterilization treatments with 70 g/pulse of a hydrogen peroxide aqueous solution (concentration: 35% by weight) and 20 pulses of aeration treatments, using the foregoing high vacuum hydrogen peroxide-sterilization device (abbreviated as "VHP Device"), storing the resulting rubber stoppers A, B and C at room temperature (23 to 25°C), and quantitatively determining the amount of the hydrogen peroxide remaining on the stoppers according to the ammonium thiocyanate method after the elapse of a predetermined period of time (immediately after the sterilization step, and 24 and 72 hours thereafter). The results thus obtained are listed in Table 2 given below and will be discussed in detail in the column entitled "Effects of the Invention" given later.

The data listed in Table 2 clearly indicates that the effects of the present invention as will be described below can be achieved in the early stage and that even if residual substances are present after the sterilization with gaseous hydrogen peroxide, the amount thereof is considerably small as compared with the remaining harmful substances observed after the EOG sterilization as will be proved in Comparative Example. In addition to the foregoing advantages, regarding the carcinogenicity of the residual substances, the difference between the hydrogen peroxide sterilization and the EOG sterilization is quite conspicuous or there is a considerable difference between the carcinogenicity of the residual substances formed after the hydrogen peroxide sterilization and that of the residual substances formed after the EOG sterilization.

Table 2:

| Results concerning the determination of the amount of hydrogen peroxide remaining on large-sized rubber stoppers | | | |
|---|---|---|---|
| | Condition: Residual Hydrogen Peroxide Conc. ($\mu$g/g) | | |
| Sample | Immediately After Sterilization | 24 Hours After Sterilization | 72 Hours After Sterilization |
| Rubber Stopper A | 0.24 | 0.14 | N.D. |
| B | 0.19 | 0.14 | N.D. |
| C | 0.28 | 0.19 | N.D. |

**Materials for rubber stoppers: chlorinated butyl rubber**

N.D.: Not detected.
The amount of the $H_2O_2$ Solution (conc.: 35% by weight) used: 70 g.
Sterilization Conditions: VHP Device was used (6 pulses of sterilization treatments + 20 pulses of aeration treatments).

Fig. 3 shows the relation between the storage time after the completion of the sterilization step and the concentration of the remaining hydrogen peroxide.

Comparative Example 1

The amount of the residual remaining on the articles after the EOG sterilization as a conventional sterilization method was determined. The results thus obtained are summarized in the following Table 3, more specifically Table 3-1, Table 3-2 and Table 3-3 (in this respect, Table 3-2 and Table 3-3 are hereunder also denoted as "Table 4") and plotted on Fig. 4, Fig. 5 and Fig. 6. In Fig. 4 and Fig. 5, the degassing treatment time (h) is plotted as abscissa and the concentration of residual sterilization agent ($\mu$g/g) is plotted as ordinate.

In this respect, the sterilization step in Table 3 comprised a sterilization treatment at 50°C for 8 hours and 4 pulses of aeration treatments; and the degassing treatment was carried out by storing in a degassing treatment chamber at 50°C and the residual gas analysis method adopted herein was GC/FID (gas chromatography/flame ionization detector) method. In addition, Table 3-1 shows the results of the determination of residual EO concentrations, and Table 3-2 shows the results of the determination of residual ECH concentrations and Table 3-3 shows those of the determination of residual EG concentrations respectively.

Table 3-1:

| Results of the determination of EO conc. remaining on rubber stoppers for syringes | | | | | | |
|---|---|---|---|---|---|---|
| Sample Name | Measuring Conditions: Time (h) After Degassing Treatment and Residual EO Conc. ($\mu$g/g) | | | | | |
| | 0 | 36 | 60 | 132 | 180 | 300 |
| 1. Top Cap | 917 | 186 | 14 | 3.6 | 3.0 | 1.8 |
| 2. Gasket | 1075 | 248 | 43 | 3.5 | 2.2 | 1.2 |
| 3. Top Gasket | 1095 | 132 | 12 | 5.5 | 3.5 | 2.4 |

**Materials for rubber stoppers: chlorinated butyl rubber.**

Sterilization agent: Ethylene oxide (EO)

Sterilization Conditions: Sterilization, 50°Cx8h + 4 Pulses of Aeration

Assembled State of Glass Syringe

Degassing Conditions: Storage within a degassing chamber (at 50°C) Method for Analyzing Residual Gas: GC/FID (Gas Chromatography/Flame Ionization Detector) Method
Top Cap: Syringe Outlet End-Sealing Tool
Gasket: Gasket for Plunger
Top Gasket (Top Packing): Gasket (packing) for sealing the joint between a resin part constituting the tip of the syringe and the syringe

Table 3-2:

| Results of the determination of ECH conc. remaining on rubber stoppers for syringes | | | | | | |
|---|---|---|---|---|---|---|
| Sample Name | Measuring Conditions: Time (h) After Degassing Treatment and Residual ECH Conc. ($\mu$g/g) | | | | | |
| | 0 | 36 | 60 | 132 | 180 | 300 |
| 1. Top Cap | 52 | 43 | 41 | 31 | 23 | 10 |
| 2. Gasket | 52 | 35 | 26 | 11 | 8.3 | 4.7 |
| 3. Top Gasket | 164 | 176 | 118 | 53 | 48 | 30 |

**Other items are identical to those specified in Table 4.**

Table 3-3:

| Results of the determination of EG conc. remaining on rubber stoppers for syringes | | | | | | |
|---|---|---|---|---|---|---|
| Sample Name | Measuring Conditions: Time (h) After Degassing Treatment and Residual EG Conc. ($\mu$g/g) | | | | | |
| | 0 | 36 | 60 | 132 | 180 | 300 |
| 1. Top Cap | 3.6 | 3.5 | 2.7 | 2.5 | 2.6 | N.D. |
| 2. Gasket | 3.4 | 3.0 | 2.8 | 2.5 | 2.6 | N.D. |
| 3. Top Gasket | 6.1 | 6.2 | N.D. | N.D. | N.D. | N.D. |

**Other items are identical to those specified in Tables 3-2 and 3-3.**

Example 2

In the case in which experiment conditions (sterilization conditions) 2-1 : 70 (g/pulse) of an aqueous hydrogen peroxide solution (a concentration of 35 % by weight) injection amount X 2 pulses and 20 pulses of an aeration were set, a sample I per sterilization bag : 20000 VFI gaskets for a syringe [formed of chlorinated butyl rubber (outer diameter 7.00 mm X overall height 6 mm)] was filled with a filling rate of 73 %, a filling rate of 53 % and a filling rate of 26 % respectively, and the sterilization bag and the basket (formed of polyethylene ; a capacity of 62 L ; an outer dimension of 622 mm X 462 mm X 268 mm) were further accommodated in parallel and experiments were variously carried out first to five times. The results of the experiments are shown in the Table 4.

The sterilization bag used herein is of a gazette type and is prepared by using a polyolefin film layer (a composition containing 50 % by weight of polyethylene and 30 % by weight of polypropylene) laminated on a polyethylene synthetic paper [trade name : Tyveck 1073B].

According to the experiment under the experimental condition 2-1, the sterilization condition was set to 70 (g/pulse) of an aqueous hydrogen peroxide solution (a concentration of 35 % by weight) injection amount X 2 pulses and 20 pulses of an aeration with a sample filling rate of 73 % in a sterilization bag 1, a sample filling rate of 53 % in a sterilization bag 2 and a sample filling rate of 26 % in a sterilization bag 3 and the treatment was carried out 1 to 5 times. As a result, a tendency of an increase in a survival ratio (a reduction in a sterilization effect) was observed with a reduction in the filling rate.

According to the experiment condition 2-1, furthermore, a survival ratio of 0/10 could not be realized in any of three kinds of filling rates. The result is shown in the Table 4.

Experiment condition 2-2 : Next, the number of pulses for the hydrogen peroxide treatment in the sterilization condition was increased from 2 to 3 under the experiment condition.

According to the experiment condition 2-2, the sterilization condition was set to 70 (g/pulse) of an aqueous hydrogen peroxide solution (a concentration of 35 % by weight) injection amount X 3 pulses and 20 pulses of an aeration with a sample filling rate of 73 % in the sterilization bag 1, a sample filling rate of 53 % in the sterilization bag 2 and a sample filling rate of 26 % in the sterilization bag 3 and the treatment was carried out 1 to 5 times. As a result, a tendency of an increase in the survival ratio (a reduction in the sterilization effect) was observed with a reduction in the filling rate.

According to the experiment condition 2-2, furthermore, it was found that the survival ratio of 0/10 can be realized for all the first to fifth treatments in the case of the filling rates of 73 % and 53 %, while the survival ratio of 0/10 cannot always be realized with the filling rate of 26 %. The result is shown in the Table 4 together.

Table 4    Sample : Result of Sterilization Treatment of VF1 rubber gasket

| Number of accommodated samples | Sterilization bag | 1 | 2 | 3 | Result of treatment and Remarks |
|---|---|---|---|---|---|
| 2 0 0 0 0 | Filling rate % | 7 3 | 5 3 | 2 6 | |
| Sterilization condition | Number of treatments | Survival Ratio | | | |
| 70 (g/pulse) x 2 pulses + aeration x 20 pulses | 1 | 0 / 1 0 | 3 / 1 0 | 2 / 1 0 | Under this treatment condition, a survival ratio of 0/10 cannot be realized irrespective of a filling rate. |
| | 2 | 3 / 1 0 | 2 / 1 0 | 5 / 1 0 | |
| | 3 | 2 / 1 0 | 0 / 1 0 | 3 / 1 0 | |
| | 4 | 1 / 1 0 | 2 / 1 0 | 1 / 1 0 | |
| | 5 | 1 / 1 0 | 4 / 1 0 | 3 / 1 0 | |
| 70 (g/pulse) x 3 pulses + aeration x 20 pulses | 1 | 0 / 1 0 | 0 / 1 0 | 0 / 1 0 | Under this treatment condition, a survival ratio of 0/10 can be always realized with a filling rate of 73 %. |
| | 2 | 0 / 1 0 | 1 / 1 0 | 0 / 1 0 | |
| | 3 | 0 / 1 0 | 0 / 1 0 | 2 / 1 0 | |
| | 4 | 0 / 1 0 | 1 / 1 0 | 1 / 1 0 | |
| | 5 | 0 / 1 0 | 0 / 1 0 | 0 / 1 0 | |

70 (g/pulse) : an aqueous hydrogen peroxide solution (concentration of 35 wt%) injection amount

Example 3

In the case in which experiment conditions (sterilization conditions) 3-1 : 70 (g/pulse) of an aqueous hydrogen peroxide solution (a concentration of 35 % by weight) injection amount X 2 pulses and 20 pulses of an aeration were set, a sample II per sterilization bag : 10000 VF3 gaskets for a syringe [formed of chlorinated butyl rubber (outer diameter 9.0 mm X overall height 8 mm)] was filled with a filling rate of 76 %, a filling rate of 53 % and a filling rate of 26 % respectively. Experiments were variously-carried out first to five times. The result of the experiments is shown in Table 5.

According to the experiment under the experiment condition 3-1, the sterilization condition was set to 70 (g/pulse) of an aqueous hydrogen peroxide solution (a concentration of 35 % by weight) injection amount X 2 pulses and 20 pulses of an aeration with a sample filling rate of 76 % in a sterilization bag 1, a sample filling rate of 53 % in a sterilization bag 2 and a sample filling rate of 26 % in a sterilization bag 3 and the treatment was carried out 1 to 5 times. As a result, a tendency of an increase in a survival ratio (a reduction in a sterilization effect) was observed with a reduction in the filling rate.

According to the experiment condition 3-1, furthermore, it was found that a survival ratio of 0/10 cannot be always realized in any of three kinds of filling rates. The result is shown in the Table 5. Experiment condition 3-2 : Next, the number of pulses for the hydrogen peroxide treatment in the sterilization condition was increased from 2 to 3 under the experiment condition.

According to the experiment condition 3-2, the sterilization condition was set to 70 (g/pulse) of an aqueous hydrogen peroxide solution (a concentration of 35 % by weight) injection amount X 3 pulses and 20 pulses of an aeration with a sample filling rate of 76 % in the sterilization bag 1, a sample filling rate of 53 % in the sterilization bag 2 and a sample filling rate of 26 % in the sterilization bag 3 and the treatment was carried out 1 to 5 times. As a result, a tendency of an increase in the survival ratio (a reduction in the sterilization effect) was observed with a reduction-in the filling rate.

According to the experiment under the experiment condition 3-2, furthermore, it was found that the survival ratio of 0/10 can be realized for all the first to fifth treatments only with the filling rate of 76 %, while the survival ratio of 0/10 cannot always be realized with the filling rates of 53 % and 26 % which are lower. The result is shown in the Table 5 together.

Table 5     Sample : Result of Sterilization Treatment of VF3 rubber

gasket

| Number of accommodated samples | Sterili- zation bag | 1 | 2 | 3 | |
|---|---|---|---|---|---|
| 1 0 0 0 0 | Filling rate % | 7 6 | 5 3 | 2 6 | |
| Sterilization condition | Number of treat- ments | Survival Ratio | | | Result of treatment and Remarks |
| 70 (g/pulse) x 2 pulses + aeration x 20 pulses | 1 | 2/.10 | 1/10 | 3/10 | Under this treatment condition, a survival ratio of 0/10 cannot be realized irrespective of a filling rate. |
| | 2 | 0/10 | 2/10 | 4/10 | |
| | 3 | 1/10 | 0/10 | 1/10 | |
| | 4 | 0/10 | 1/10 | 3/10 | |
| | 5 | 1/10 | 1/10 | 0/10 | |
| 70 (g/pulse) x 3 pulses + aeration x 20 pulses | 1 | 0/10 | 0/10 | 0/10 | Under this treatment condition, a survival ratio of 0/10 can be always realized with a filling rate of 76 %. |
| | 2 | 0/10 | 0/10 | 0/10 | |
| | 3 | 0/10 | 0/10 | 1/10 | |
| | 4 | 0/10 | 1/10 | 1/10 | |
| | 5 | 0/10 | 0/10 | 0/10 | |

70 (g/pulse) : an aqueous hydrogen peroxide solution (concentration of

35 wt%) injection amount

Example 4

In the case in which experimental conditions (sterilization conditions) 4-1 : 70 (g/pulse) of an aqueous hydrogen peroxide solution (a concentration of 35 % by weight) injection amount X 2 pulses and 20 pulses of an aeration were set, a sample III per sterilization bag : 4000 VF5 gaskets for a syringe [formed of chlorinated butyl rubber (diameter 12.0 mm X overall height 10 mm)] was filled with a filling rate of 73 %, a filling rate of 53 % and a filling rate of 26 % respectively. Experiments were variously carried out first to five times. The result of the experiments is shown in Table 6.

According to the experiment condition 4-1, the sterilization condition was set to 70 (g/pulse) of an aqueous hydrogen peroxide solution injection amount X 2 pulses and 20 pulses of an aeration 20 with a sample filling rate of 73 % in a sterilization bag 1, a sample filling rate of 53 % in a sterilization bag 2 and a sample filling rate of 26 % in a sterilization bag 3 and the treatment was carried out 1 to 5 times. As a result, a tendency of an increase in a survival ratio (a reduction in a sterilization effect) was observed with a reduction in the filling rate.

According to the experiment under the experiment condition 4-1, furthermore, it was found that a survival ratio of 0/10 cannot be always realized in any of three kinds of filling rates. The result is shown in the Table 6.

Experiment condition 4-2 : Next, the number of pulses for the hydrogen peroxide treatment in the sterilization condition was increased from 2 to 3 under the experiment condition.

According to the experiment under the experiment condition 4-2, the sterilization condition was set to 70 (g/pulse) of an aqueous hydrogen peroxide solution (a concentration of 35 % by weight) injection amount X 3 pulses and 20 pulses of an aeration with a sample filling rate of 73 % in the sterilization bag 1, a sample filling rate of 53 % in the sterilization bag 2 and a sample filling rate of 26 % in the sterilization bag 3 and the treatment was carried out 1 to 5 times. As a result, a tendency of an increase in the survival ratio (a reduction in the sterilization effect) was observed with a reduction in the filling rate.

According to the experiment under the experiment condition 4-2, furthermore, it was found that the survival ratio of 0/10 can be realized for all the first to fifth treatments with the filling rates of 73 % and 53 %, while the survival ratio of 0/10 cannot always be realized with the filling rate of 26 % which is much lower. The result is shown in the Table 6 together.

Table 6   Result of Sterilization Treatment of VF5

gasket

| Number of accommodated samples | Sterili- zation bag | 1 | 2 | 3 | Result of treatment and Remarks |
|---|---|---|---|---|---|
| 4 0 0 0 | Filling rate % | 7 3 | 5 3 | 2 6 | |
| Sterilization condition | Number of treat- ments | Survival Ratio | | | |
| 70 (g/pulse) x 2 pulses + aeration x 20 pulses | 1 | 1 / 1 0 | 1 / 1 0 | 2 / 1 0 | Under this treatment condition, a survival ratio of 0/10 cannot be realized irrespective of a filling rate. |
| | 2 | 0 / 1 0 | 1 / 1 0 | 1 / 1 0 | |
| | 3 | 2 / 1 0 | 0 / 1 0 | 3 / 1 0 | |
| | 4 | 0 / 1 0 | 1 / 1 0 | 0 / 1 0 | |
| | 5 | 0 / 1 0 | 2 / 1 0 | 1 / 1 0 | |
| 70 (g/pulse) x 3 pulses + aeration x 20 pulses | 1 | 0 / 1 0 | 0 / 1 0 | 0 / 1 0 | Under this treatment condition, a survival ratio of 0/10 can be always realized with a filling rate of 73 %. |
| | 2 | 0 / 1 0 | 0 / 1 0 | 0 / 1 0 | |
| | 3 | 0 / 1 0 | 0 / 1 0 | 0 / 1 0 | |
| | 4 | 0 / 1 0 | 0 / 1 0 | 0 / 1 0 | |
| | 5 | 0 / 1 0 | 0 / 1 0 | 1 / 1 0 | |

70 (g/pulse) : an aqueous hydrogen (concentration of 35 wt%)

injection amount

Example 5

In the case in which experiment conditions (sterilization conditions) 5-1 : 70 (g/pulse) of an aqueous hydrogen peroxide solution (a concentration of 35 % by weight) injection amount X 2 pulses and 20 pulses of an aeration were set, a sample IV per sterilization bag : 150 rubber gaskets for a large-sized syringe [formed of chlorinated butyl rubber (diameter 42.0 mm X overall height 36 mm)] was filled with a filling rate of 73 %, a filling rate of 51 % and a filling rate of 23 % respectively. Experiments were variously carried out first to five times.

According to the experiment under the experiment condition 5-1, the sterilization condition was set to 70 (g/ pulse) of an aqueous hydrogen peroxide solution (a concentration of 35 % by weight) injection amount X 2 pulses and 20 pulses of an aeration with a sample filling rate of 73 % in a sterilization bag 1, a sample filling rate of 51 % in a sterilization bag 2 and a sample filling rate of 23 % in a sterilization bag 3 and the treatment was carried out 1 to 5 times. As a result, a survival ratio of 0/10 was observed with any filling rate any number of times. The result of the experiment is shown in Table 7.

Experiment condition 5-2 : Next, the number of pulses for the hydrogen peroxide treatment in the sterilization condition was increased from 2 to 3 under the experiment condition.

According to the experiment under the experiment condition 5-2, the sterilization condition was set to 70 (g/ pulse) of an aqueous hydrogen peroxide solution (a concentration of 35 % by weight) injection amount X 3 pulses and 20 pulses of an aeration with a sample filling rate of 73 % in the sterilization bag 1, a sample filling rate of 51 % in the sterilization bag 2 and a sample filling rate of 23 % in the sterilization bag 3 and the treatment was carried out 1 to 5 times. As a result, a survival ratio 0/10 could be realized with any filling rate any number of times. The result of the experiment is shown in the table 7 together.

Table 7　　Sample : Result of Sterilization Treatment of rubber

gasket for large-sized syringe

| Number of accommodated samples | Sterili- zation bag | 1 | 2 | 3 | |
|---|---|---|---|---|---|
| 1 5 0 | Filling rate % | 7 3 | 5 1 | 2 3 | |
| Sterilization condition | Number of treat- ments | Survival Ratio | | | Result of treatment and Remarks |
| 70 (g/pulse) x 2 pulses + aeration x 20 pulses | 1 | 0 / 1 0 | 0 / 1 0 | 0 / 1 0 | Under this treatment condition, a survival ratio of 0/10 can be always realized irrespective of a filling rate. |
| | 2 | 0 / 1 0 | 0 / 1 0 | 0 / 1 0 | |
| | 3 | 0 / 1 0 | 0 / 1 0 | 0 / 1 0 | |
| | 4 | 0 / 1 0 | 0 / 1 0 | 0 / 1 0 | |
| | 5 | 0 / 1 0 | 0 / 1 0 | 0 / 1 0 | |
| 70 (g/pulse) x 3 pulses + aeration x 20 pulses | 1 | 0 / 1 0 | 0 / 1 0 | 0 / 1 0 | Under this treatment condition, a survival ratio of 0/10 can be always realized irrespective of a filling rate. |
| | 2 | 0 / 1 0 | 0 / 1 0 | 0 / 1 0 | |
| | 3 | 0 / 1 0 | 0 / 1 0 | 0 / 1 0 | |
| | 4 | 0 / 1 0 | 0 / 1 0 | 0 / 1 0 | |
| | 5 | 0 / 1 0 | 0 / 1 0 | 0 / 1 0 | |

70 (g/pulse) : an aqueous hydrogen peroxide solution (concentration of

35 wt%) injection amount

Example 6

There was investigated the influence, on a sterilization efficiency, of a ratio of a capacity of a sterilization bag to a capacity of a porous container (basket) accommodating the sterilization bag (former / latter = volume rate) in sterilization using gaseous hydrogen peroxide. A material, an outer dimension and an inner volume of a basket :

- Basket 1 : formed of PE ; outer dimension 622 mm X 462 mm X 196 mm ; inner volume 45 L ;
- Basket 2 : formed of PE ; outer dimension 622 mm X 462 mm X 268 mm ; inner volume 62 L ;
- Basket 3 : formed of PE ; outer dimension 820 mm X 570 mm X 428 mm ; inner volume 161 L .

In the case in which treatment conditions (sterilization conditions) : 70 (g/pulse) of an aqueous hydrogen peroxide solution (a concentration of 35 % by weight) injection amount X 3 pulses and 20 pulses of an aeration were set, a sample IV per sterilization bag : 5000 VF5 gaskets for a syringe [formed of chlorinated butyl rubber (diameter 12.0 mm X overall height 10 mm)] was filled with a filling rate of 67 %, a filling rate of 48 % and a filling rate of 16 % respectively. Experiments were variously carried out first to five times. The result of the experiments is shown in Table 8.

According to the experiment of the sixth embodiment, a survival ratio of 0/10 could not be always realized with a maximum volume rate of 67 %, while a survival ratio of 0/10 could be realized with the volume rates of 48 % and 16 % which are lower at first to fifth times of the treatment.

Table 8

| Result of sterilization treatment for basket selection | | | | | |
|---|---|---|---|---|---|
| Basket number to be used together | Capacity (L) | Volume rate % | Number of treatments | Survival Ratio % | Remarks |
| 1 | 4 5 | 6 7 | 1 | 1/1 0 | |
| Ditto | Ditto | Ditto | 2 | 0/1 0 | |
| Ditto | Ditto | Ditto | 3 | 0/1 0 | |
| Ditto | Ditto | Ditto | 4 | 1/1 0 | |
| Ditto | Ditto | Ditto | 5 | 0/1 0 | |
| 2 | 6 2 | 4 8 | 1 | 0/1 0 | |
| Ditto | Ditto | Ditto | 2 | 0/1 0 | |
| Ditto | Ditto | Ditto | 3 | 0/1 0 | |
| Ditto | Ditto | Ditto | 4 | 0/1 0 | |
| Ditto | Ditto | Ditto | 5 | 0/1 0 | |
| 3 | 1 6 1 | 1 6 | 1 | 0/1 0 | A sterilization bag is broken due to rapid expansion. |
| Ditto | Ditto | Ditto | 2 | 0/1 0 | |
| Ditto | Ditto | Ditto | 3 | 0/10 | |
| Ditto | Ditto | Ditto | 4 | 0/1 0 | |
| Ditto | Ditto | Ditto | 5 | 0/1 0 | |

Effects of the Invention

The sterilization method according to the present invention and a variety of sealing tools obtained using the method show various effects detailed below:

(1) Immediately after the completion of the sterilization step, the residual amounts of hydrogen peroxide were found to be 0.24μg/g for the rubber stopper A, 0.19μg/g for the rubber stopper B and 0.28 μg/g for the rubber stopper C, all of which are much lower than a normal value of 0.5μg/g. These results clearly indicate that the rubber stoppers A, B and C can be used without problems.

(2) Any residual hydrogen peroxide cannot be detected in all of the rubber stoppers after 72 hours from the completion of the sterilization step (or the amount thereof is less than the detection limit). As has been described in the foregoing item (1) and (2), the residual amount of hydrogen peroxide is reduced to the level, which is out of the question or is practically acceptable after 24 hours from the completion of the sterilization step and therefore, these results simply show the time required for the reduction of the residual amount to the level of less than the detection limit.

(3) The time required for the sterilization treatment is on the order of about 1.5 hour per batch for an ophthalmic medicaments nozzle, a vial for ophthalmic medicaments or the like and this time is sufficient to ensure the degree of sterilization of SAL = 10EXP(-6).

(4) The amounts of hydrogen peroxide remaining on the containers of polyethylene (PE), polypropylene (PP) or polytetrafluoroethylene (PTFE) after the sterilization can be improved to a level of about 0.2 to 0.3 ppm immediately after the sterilization, a level of 0.1 to 0.2 ppm one day after the sterilization and a level of not more than the detection limit (0.1 ppm) one week after the sterilization.

Therefore, these containers can be used immediately after the sterilization treatment.

(5) It is not necessary to remove, for instance, moisture and/or organic solvent from the article after the sterilization treatment or the article further subjected to the aeration treatment before forwarding.

(6) If a filling rate in a sterilization bag for a sample is set to be high, a survival ratio can be reduced (a sterilization effect can be increased).

(7) If a filling rate of 53 % or more is set to be combined with a great sterilization condition of "70 (g/pulse) of an aqueous hydrogen peroxide solution (a concentration of 35 % by weight) injection amount X 3 pulses and 20 pulses of an aeration", a survival ratio of 0/10 can be always realized in any of the second to sixth embodiments.
(8) If a ratio of the content of a basket accommodated in a sterilizer in a stacking process to an outer volume of an outer bag mounted thereon (latter / former ; volume rate) is set to be 48 % or less, a survival ratio of 0/10 can be always realized.
(9) A polyolefinic material is optimum for the sterilization bag and a second optimum material could not be found.

**Claims**

1. A method for the production of a sealing tool which comprises setting a filling rate in a sterilization bag for a sample to be sterilized to 45 % or more for a gasket for a normal type syringe and to 20 % or more for a gasket for a large-sized syringe, thereby carrying out sterilization in the case in which a rubbery sealing tool or a sealing tool made from an olefinic resin is held as an article to be processed in a sterilization unit under a high vacuum, gaseous hydrogen peroxide is then introduced into the sterilization unit, and is held for a predetermined time and is sterilized by at least one member selected from the group consisting of active oxygen and radical hydroxide, a clean gas is thereafter introduced and is held for a predetermined time to cause a sterilizing substance to penetrate into an inner side of the article to be processed, thereby setting a sterilizing condition using the gaseous hydrogen peroxide in which a sterilization treatment for the article to be processed is one sterilization pulse to a combination of 70 (g/pulse) of an aqueous hydrogen peroxide solution (a concentration of 35 % by weight) injection amount X 3 pulses and 20 pulses of an aeration.

2. The method for the production of a sealing tool as set forth in claim 1, which comprises setting the filling rate of the sample to be sterilized in the sterilization bag to be to 50 % or more for a gasket for a normal type syringe and to 20 % or more for a gasket for a large-sized syringe under the sterilization condition, thereby carrying out the sterilization.

3. A method for the production of a sealing tool, which comprises setting a sterilization condition to a combination of 70 (g/pulse) of an aqueous hydrogen peroxide solution (a concentration of 35 % by weight) injection amount X 4 pulses and 20 pulses of an aeration, setting a filling rate of a sample to be sterilized in a sterilization bag to be to 20 % or more for a gasket for a normal type syringe or a gasket for a large-sized syringe, thereby carrying out the sterilization.

4. The method for the production of a sealing tool as set forth in any one of claims 1 to 3, which comprises setting the number of repetitions of the aeration pulse to be carried out next to a sterilization pulse in the sterilization treatment to be 30 pulses or more.

5. The method for the production of a sealing tool as set forth in any one of claims 1 to 3, which comprises setting the number of repetitions of the aeration pulse to be 5 to 50 pulses or more.

6. The method for the production of a sealing tool as set forth in any one of claims 1 to 4, which comprises setting the sterilization pulses and the aeration pulses to be, in advance, conducted in combination.

7. The method for the production of a sealing tool as set forth in any one of claims 1 to 5, which comprises setting an outer bag further accommodating the sterilization bag having the article to be processed to be mounted in a porous container for mounting with a volume rate of 12 to 55 %, thereby carrying out the sterilization treatment.

8. The method for the production of a sealing tool as set forth in any one of claims 1 to 5, which comprises setting the article to be processed to be at least one member selected from a rubber cap, a rubber gasket, a gasket for a piston (plunger) to be inserted into an injection cylinder (syringe), a tool for preventing liquid leakage such as rubber boots, and an elastic ring for a bushing and for fitting a joint.

9. The method for the production of a sealing member as set forth in any one of claims 1 to 7, which comprises rubber being at least one member selected from the following conjugated diene rubber and non-conjugated diene rubber: the conjugated diene rubber being natural rubber, a variety of synthetic rubber materials, blends each comprising at least two of these natural and synthetic rubber materials and copolymer rubber comprising repeating units of these rubber materials and other repeating units copolymerizable therewith, wherein the synthetic rubber comprises 1,4-cis-polyisoprene rubber obtained by 1,4-addition polymerization of isoprene, which is a repeating unit mainly constituting the natural rubber, 1,4-cis-polybutadiene rubber and isobutene-isoprene copolymer rubber; the non-conjugated diene rubber being copolymer rubber materials of at least two 1-olefins or multi-component copolymer rubber materials obtained by copolymerizing these monomers with third non-conjugated dienes, wherein the copolymer rubber materials of at least two 1-olefins is at least one member selected from the group consisting of ethylene-propylene (copolymer) rubber, ethylene-1-butene copolymer rubber and propylene-1-butene copolymer rubber, and wherein the multi-component copolymer rubber obtained by copolymerizing these monomers with a third non-conjugated diene is at least one member selected from the group consisting of ethylene-propylene-1,4-hexadiene copolymer rubber, ethylene-propylene-methylene norbornene copolymer rubber and ethylene-propylene-ethylidene norbornene copolymer rubber.

10. The method for the production of a sealing tool as set forth in any one of claims 1 to 8, which comprises the thermoplastic elastomer (thermoplastic rubber) being a polymer or a kneaded composition (kneaded mixture) of at least two polymers, which simultaneously has characteristic properties of thermoplastic resin and elastomer; the polymer composition, which can be formed into a variety of shapes as set forth in the molding method applicable to the resin and can be subjected to vulcanization treatment (crosslinking treatment) applicable to the elastomer, is at least one kneaded composition selected from the group consisting of kneaded compositions of polyolefin resins and ethylene-propylene (copolymer) rubber, kneaded compositions of polyolefin resins and ethylene-propylene-non-conjugated diene copolymer rubber and kneaded compositions of propylene-1-butene copolymer resins and ethylene-propylene-non- conjugated diene copolymer rubber.

11. The method for the production of a sealing tool as set forth in any one of claims 1 to 9, which comprises the thermoplasticelastomer being a thermally kneaded composition comprising at least one member selected from the group consisting of polyethylene resins, polypropylene resins, poly-1-butene resins, poly-4-methyl-1-pentene resins and poly-1-hexene resins; and at least one member selected from the group consisting of ethylene-propylene-1,4-hexadiene copolymer rubber, ethylene-propylene-methylene norbornene copolymer rubber and ethylene-propylene-ethylidene norbornene copolymer rubber.

## Fig. 1

EP 1 283 061 A1

# Fig. 2

Outlined Process for Sterilization Cycle

Drying Phase
(2 pulses)    Leakage Test    Sterilization Phase (4 pulses)    Aeration Phase (20 pulses)

Pressure axis labels:
93.3kPa (700Torr)
80.0kPa (600Torr)
68.7kPa (500Torr)
53.2kPa (400Torr)
40.0kPa (300Torr)
0.67kPa (5Torr)

Pressure

Time    (10 min/scale)

In case where the sterilization cycle comprises a combination
of 4 pulses of gaseous hydrogen peroxide treatment and 20 aeration pulses,
the sterilization process requires about 3 hours and 20 minutes.

*Fig. 3*

Correlation between the storage time and
the residual hydrogen peroxide concentration

EP 1 283 061 A1

Fig. 4

Time required for the degassing treatment and residual EO concentration

Legend:
- Top Cap
- Gasket
- Top Gasket

X-axis: Time Required for Degassing Treatment (hr.)

Y-axis: Residual EO Concentration ($\mu$g/g)

EP 1 283 061 A1

*Fig. 5*  Time required for the degassing treatment and residual EG concentration

Legend:
- ■ Top Cap
- ✕ Gasket
- ● Top Gasket

Y-axis: Residual EG Concentration ($\mu$g/g)

X-axis: Time Required for Degassing Treatment (hr.)

EP 1 283 061 A1

*Fig. 6*

Time required for the degassing treatment and residual ECH concentration

EP 1 283 061 A1

## INTERNATIONAL SEARCH REPORT

| International application No. |
| --- |
| PCT/JP01/04039 |

| A. CLASSIFICATION OF SUBJECT MATTER |
| --- |
| Int.Cl$^7$   A61L2/20, A61L2/26, B65B55/04 |

According to International Patent Classification (IPC) or to both national classification and IPC

| B. FIELDS SEARCHED |
| --- |
| Minimum documentation searched (classification system followed by classification symbols)<br>Int.Cl$^7$   A61L2/16-2/22, A61L2/26, A61L31/00, B65B55/02-55/10 |

Documentation searched other than minimum documentation to the extent that such documents are included in the fields searched
| Jitsuyo Shinan Koho | 1926-1996 | Toroku Jitsuyo Shinan Koho | 1994-2001 |
| Kokai Jitsuyo Shinan Koho | 1971-2001 | Jitsuyo Shinan Toroku Koho | 1996-2001 |

Electronic data base consulted during the international search (name of data base and, where practicable, search terms used)

| C. DOCUMENTS CONSIDERED TO BE RELEVANT | | |
| --- | --- | --- |
| Category* | Citation of document, with indication, where appropriate, of the relevant passages | Relevant to claim No. |
| Y | JP 8-505786 A (American Sterilizer Company), 25 June, 1996 (25.06.96), page 17, lines 10, 16; page 18, lines 13 to 22 & WO 94/11034 A1   & US 5837193 A & EP 668783 A | 1-11 |
| Y | JP 2-4624 A (Japan Crown Cork Co., Ltd.), 09 January, 1990 (09.01.90), page 4, upper left column, line 17 to page 5, upper left column, line 17 (Family: none) | 1-11 |
| Y | JP 11-193010 A (Seikagaku Corporation), 21 July, 1999 (21.07.99), Full text; all drawings & WO 99/27971 A2   & EP 971749 A | 1-11 |
| Y | JP 64-25865 A (Iwasaki Electric Co., Ltd.), 27 January, 1989 (27.01.89), Full text; Fig. 2   (Family: none) | 7,9-11 |

| ☒ | Further documents are listed in the continuation of Box C. | ☐ | See patent family annex. |
| --- | --- | --- | --- |

| * | Special categories of cited documents: |
| --- | --- |
| "A" | document defining the general state of the art which is not considered to be of particular relevance |
| "E" | earlier document but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search<br>07 August, 2001 (07.08.01) | Date of mailing of the international search report<br>21 August, 2001 (21.08.01) |
| --- | --- |
| Name and mailing address of the ISA/<br>   Japanese Patent Office | Authorized officer |
| Facsimile No. | Telephone No. |

Form PCT/ISA/210 (second sheet) (July 1992)

33

**EP 1 283 061 A1**

## INTERNATIONAL SEARCH REPORT

| | International application No. |
|---|---|
| | PCT/JP01/04039 |

C (Continuation). DOCUMENTS CONSIDERED TO BE RELEVANT

| Category* | Citation of document, with indication, where appropriate, of the relevant passages | Relevant to claim No. |
|---|---|---|
| Y | JP 62-176455 A (Kawasumi Lab Inc.),<br>03 August, 1987 (03.08.87),<br>page 3, upper right column, line 16 to page 4, upper left column, line 1; page 5, table 1; page 6, table 2<br>(Family: none) | 9 |
| Y | JP 58-58057 A (Terumo Corporation),<br>06 April, 1983 (06.04.83),<br>page 3, upper right column<br>& BE 894575 A1  & GB 2108943 A<br>& US 4444330 A  & FR 2542612 A | 10 |
| Y | JP 6-327760 A (Japan Synthetic Rubber Co., Ltd.),<br>29 November, 1994 (29.11.94),<br>Par. No. [0035]<br>(Family: none) | 11 |

Form PCT/ISA/210 (continuation of second sheet) (July 1992)